# EUROPEAN PATENT SPECIFICATION

(11) **EP 4 249 004 B1**
(45) Date of publication and mention of the grant of the patent: **14.01.2026**
(21) Application number: 22742158.3
(22) Date of filing: 18.01.2022
(51) Int. Cl.: A61K 51/04, A61P 35/00, A61P 35/04, A61K 103/30, A61K 103/32, A61K 103/00

(54) **DZ-1-LYS-DOTA CONJUGATE COMPOUNDED WITH RADIOACTIVE METAL AND APPLICATION**
DZ-1-LYS-DOTA-KONJUGAT, ZUSAMMENGESETZT MIT RADIOAKTIVEM METALL UND ANWENDUNG
CONJUGUÉ DZ-1-LYS-DOTA MÉLANGÉ À UN MÉTAL RADIOACTIF ET APPLICATION

(30) Priority: 19.01.2021 CN 202110070726
(43) Date of publication of application: 27.09.2023
(73) Proprietor: Beijing Jianshu Medical Technology Co., Ltd., Beijing 110101 (CN)
(72) Inventor: CHUNG, Lelandwei-Kwo, Chengdu, Sichuan 610041 (CN); WANG, Ruoxiang, Chengdu, Sichuan 610041 (CN); ZHANG, Yi, Chengdu, Sichuan 610041 (CN); CHENG, Yuping, Chengdu, Sichuan 610041 (CN); YU, Bo, Chengdu, Sichuan 610041 (CN)
(74) Representative: Moré, Solveig Helga
(86) International application number: PCT/CN2022/072567
(87) International publication number: WO 2022/156679

(56) References cited:
- WO-A1-2022/147224
- CN-A- 104 312 194
- CN-A- 112 870 389
- CN-A- 113 105 461
- US-A1- 2014 248 213
- US-A1- 2014 248 213
- POOT ALEX J. ET AL: "The Current Status and Future Potential of Theranostics to Diagnose and Treat Childhood Cancer", FRONTIERS IN ONCOLOGY, vol. 10, 19 November 2020 (2020-11-19), XP093209605, ISSN: 2234-943X, DOI: 10.3389/fonc.2020.578286
- WEINEISEN MARTINA ET AL: "68 Ga- and 177 Lu-Labeled PSMA I&T: Optimization of a PSMA-Targeted Theranostic Concept and First Proof-of-Concept Human Studies", vol. 56, no. 8, 18 June 2015 (2015-06-18), US, pages 1169 - 1176, XP055919654, ISSN: 0161-5505, Retrieved from the Internet <URL:https://jnm.snmjournals.org/content/jnumed/56/8/1169.full.pdf> [retrieved on 20240927], DOI: 10.2967/jnumed.115.158550
- JASON BOYANG WU ET AL: "Near-infrared fluorescence heptamethine carbocyanine dyes mediate imaging and targeted drug delivery for human brain tumor", BIOMATERIALS, vol. 67, 16 July 2015 (2015-07-16), AMSTERDAM, NL, pages 1 - 10, XP055480650, ISSN: 0142-9612, DOI: 10.1016/j.biomaterials.2015.07.028
- REICHEL DEREK ET AL: "Near Infrared Fluorescent Nanoplatform for Targeted Intraoperative Resection and Chemotherapeutic Treatment of Glioblastoma", ACS NANO, vol. 14, no. 7, 17 June 2020 (2020-06-17), US, pages 8392 - 8408, XP093208903, ISSN: 1936-0851, DOI: 10.1021/acsnano.0c02509
- XIAO L, ZHANG Y, YUE W, XIE X, WANG J P, CHORDIA M D, CHUNG L W, PAN D.: "Heptamethine cyanine based (64)Cu-PET probe PC-1001 for cancer imaging: synthesis and in vivo evaluation.", NUCL. MED. BIOL., ELSEVIER, NY., US, vol. 40, no. 3, 1 April 2013 (2013-04-01), US , pages 351 - 360, XP002727409, ISSN: 0969-8051, DOI: 10.1016/j.nucmedbio.2013.01.001

## Description

### TECHNICAL FIELD

The invention relates to the technical field of DZ-1-Lys-DOTA conjugates, in particular to a pharmaceutical composition comprising complexes of a DZ-1-Lys-DOTA conjugate with radioactive metals and applications.

### BACKGROUND

A limited number of diagnostic or therapeutic radiopharmaceuticals are available for imaging or treating cancer or tumors. Imaging utilizes very low concentrations required for detection, such as positron emission tomography (PET) detection of positron emitters. Internal radiation therapy is a treatment method in which a radioactive source is placed inside a subject for cancer treatment. Internal radiation therapy can be systemic, in which it travels with the blood into all body tissues, killing cancer cells through radiation to cells near the radioactive metal. Radioactive metals can be delivered through chelation or complexation with a variety of chelating compounds and coupling to targeting ligands.

Various targeting ligands, chelators, and radioactive combinations have been attempted for use in imaging, radiotherapy, or both, including therapeutic approaches that pair the same targeting ligands. However, the affinity of targeting ligands for their receptors has been found to be strongly dependent both on the chelator and radioactive metal used, so their efficacy is unpredictable. Similarly, unpredictability applies to the stability of radioactive metal complexes, as retention of radioactive metals may be affected by targeting ligands, chelators, or combinations thereof, as well as their combination with the desired radioactive metal. A particular problem with paired therapy using the same pharmacophore (different radioactive metal but the same carrier) is that the reporter gene (imaging agent) and the therapeutic should show very similar pharmacokinetics to be suitable for paired use, but this may not be the case, as individual metal chelator conjugates may have significant effects on affinity and/or stability.

Currently, the radionuclide complexes used for such targeted radiotherapy are mostly diagnostic with limited therapeutic applications. The targeting ligands include various antibodies, proteins and some smaller compounds, especially polypeptides that can target certain types of cancer.

Radionuclides and radioactive metals are more used for tumor imaging or for adding imaging capabilities to targeted cancer drugs as opposed to their use in radiotherapy. Certain dye conjugates are known from WO 2016106324 and US 10,307,489 for targeted cancer drug therapy and imaging, which include two specific double-coupled dye-drug-chelator conjugates, wherein the dye is combined with a) anticancer drug gemcitabine and b) the imaging group coupling, wherein the imaging group is ⁶⁴Cu-DOTA radioactive metal or Gd³⁺-DOTA non-radioactive metals. Chelated/complexed radioactive metals are used to provide imaging capabilities for cancer drug conjugates.

US 2014/248213 A1 discloses a Dual Nuclear/NIR Agent having the following structure:

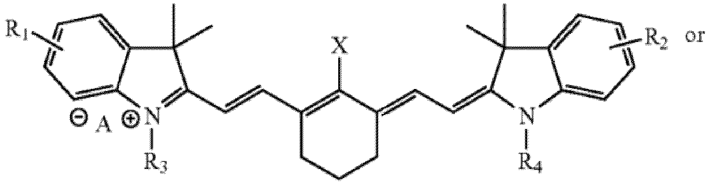

This agent encompasses compounds comprising a heptamethine cyanine dye conjugated to a chelator labelled with a metal selected from ⁶⁴Cu, ¹¹¹In, ^{99m}Tc, ⁶⁸Ga, ⁹⁰Y and ¹⁷⁷Lu.

Similarly, a lysine-crosslinked heptamethyl carbocyanine dye complexed with DOTA radiochemical Cu-64 (DZ-1-Lys-DOTA-Cu-64) conjugates for cancer imaging described by Xiao et al. in Nuclear Medicine and Biology 40, p. 351-360 (2013).

A known problem with radioactive metal complexes can be the lack of stability in vivo (e.g., blood and/or serum), which may result in insufficient performance, especially for the imaging performance in the attempts to detect small secondary tumors or metastases, as well as inefficiencies and unnecessarily high radiation exposure.

Radiation therapy tends to be more challenging than imaging alone, so options for treatment with radiation therapy are more limited. Appropriate radionuclides need to be carefully combined with appropriate chelators and appropriate targeting ligands to provide the desired properties, including affinity, stability, and pharmacokinetics. A specific radioactive metal may emit one or more types of particles or radioactive species at specific different percentages, and for proper targeting, careful coordination of the carrier with a given radioactive metal is required to keep the complex stable enough to effectively destroy the target cancer, but causes no collateral radiation damage to the rest of the body and its organs and tissues when arriving at the target site.

Some therapies can provide both therapeutic and diagnostic imaging to some extent, although theranostic agents are often employed in order to improve the performance of each therapeutic agent. "True" theranostic agents are identical pairs, i.e., have the same carrier for the theranostic radioactive metal to destroy tumors, and the imaging radioactive metals are used for diagnosis and/or guide therapy.

However, both chelators and radioactive metals affect important performance characteristics and pharmacokinetics, so a given carrier may not provide the same pairing with suitable characteristics, stability, and efficacy for the desired combination of radioactive metals. As an alternative, if there are no clinically significant differences in binding, non-similar pairings can be used to adjust features.

An example of the use of this peptide radiopharmaceutical dissimilar pairing for the treatment of somatostatin receptor (SSTR) positive neuroendocrine tumors (NETs) is the use of Lu-177-DOTA-TATE with Ga-68-DOTA-TOC, each polypeptide ligand binds to tumor cells with different affinities, but the difference is said to have no clinical significance.

A particular problem with radiochemical is the intense and persistent localization of radioactivity observed in certain organs such as, but not limited to, the kidney, which compromises tumor visualization in the kidney area and limits therapeutic potential, especially for the kidney tumors and tumors localized in this area. It is therefore beneficial to reduce the level of radioactivity in the kidney, but not in the target tissue. Radionuclide complexes may also encounter renal proximal tubule reabsorption and/or unfavorable prolonged retention of radio metabolites in cells, especially kidney cells or cells of other organs, which may lead to undesired persistent radioactivity.

Other problems with radiochemical may include one or more of the following: lack of applicability for one or more cancer treatments, insufficient sensitivity, insufficient selectivity for cancer cells, insufficient contrast due to changes over time between tumor and non-cancer tissue, insufficient biodistribution, insufficient tumor targeting, insufficient tissue and/or tumor penetration, uneven distribution in tumor tissue, insufficient tumor accumulation rate, insufficient retention in tumor, and slow clearance from blood after administration, low radiation exposure of tissues and organs, low clearance rate of vital organs such as kidney, liver, heart, etc., non-target tissues or organs, especially cystic tissues, such as organs and dense tissues, including tumor tissues, especially calcified of tumor tissue, slowly establishing steady state distribution in the body and organs including the heart, liver, lung and kidney, persistent radioactivity in one or more organs including the kidney and/or liver, failure to or difficult to form stable complex with one or more radioactive metals, low complex formation rate, low labeling efficiency with one or more radioactive metals, insufficient solubility in aqueous solutions, low stability in aqueous solutions, low stability at physiological pH, low in vivo stability, high dissociation rate between radioactive metals and complexes (especially high dissociation rate in vivo), slow tumor-specific targeting, and high organ accumulation (such as kidney, spleen, liver, Heart), slow clearance (such as from blood, liver, kidney and other organs), difficulty in synthesis or high cost.

Therefore, there is an urgent need to provide theranostic radiopharmaceuticals that improve imaging, improve therapy, or both. It requires better matched therapy pairs with the same affinity, sufficient efficacy and stability, and provides improved image-guided therapy.

### SUMMARY OF THE INVENTION

In view of the above-mentioned problems, the object of the present invention is to provide DZ-1-Lys-DOTA conjugates complexed with radioactive metals and use thereof. The present invention is set out in the appended set of claims.

In particular, the present invention provides a pharmaceutical composition comprising a first complex and a second complex of DZ-1-Lys-DOTA conjugate with a radioactive metal M, wherein the DZ-1-Lys-DOTA conjugate comprises a heptamethyl carbocyanine dye coupled with a DOTA group via a lysine cross-linking agent, and the molecular formula of the complex is as follows: 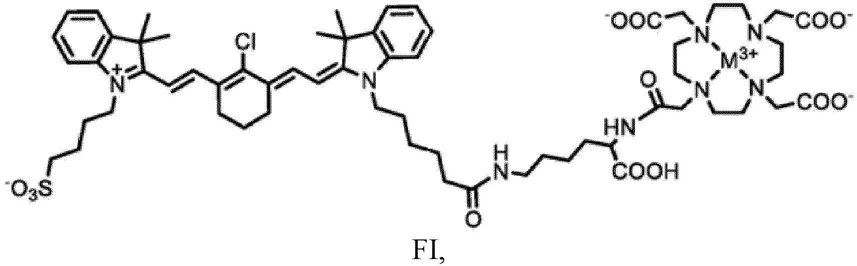 wherein the DOTA group is complexed with the radioactive metal M and wherein the radioactive metal Min the first complex is gallium-68, and the radioactive metal M in the second complex is lutetium-177 or yttrium-90.

The pharmaceutical composition may contain 0.01% to 99% of the first complex and the second complex.

The pharmaceutical composition may further comprise one or more pharmaceutical excipients.

The complex may be formed by mixing and complexing the DZ-1-Lys-DOTA conjugate with the radioactive metals, and with one or more reagents, buffers or excipients;
wherein the DZ-1-Lys-DOTA conjugate has the molecular structure of formula FII:

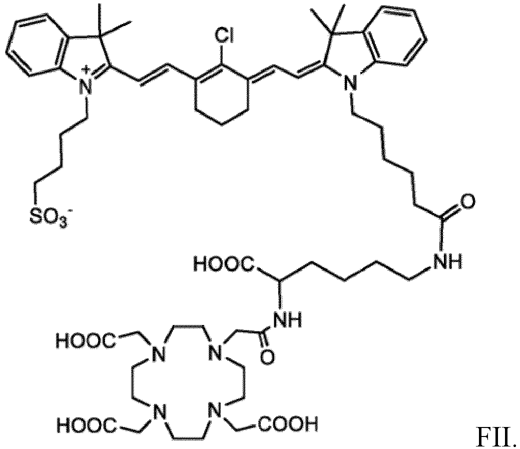

The pharmaceutical composition may be for use in the image-guided therapy of cancer, wherein the first complex and the second complex of the DZ-1-Lys-DOTA conjugate having a molecular structure FI are co-administered to a subject with cancer. The cancer may comprise precancerous and cancer cells or tissues, tumors and metastatic tumors thereof, primary and secondary tumors. In particular, it may comprise brain cancer, brain tumors and metastatic tumors thereof.

The treatment of the cancer may be a radiation therapy. In such a scenario, a radioactive amount of the complex during the radiation therapy may be less than 50 µCi. A dose of the complex may be less than 250 kBq/kg in the radiation therapy.

The radiation therapy may be paired with positron emission tomography or SPECT and/or may provide image-guided therapy.

The route of administration of the pharmaceutical composition of the present invention may be oral, intravenous, interstitial, intraperitoneal, subcutaneous or intramuscular injection, and/or by brachytherapy.

Compared with the prior art, the present invention has the following beneficial effects.

The present invention includes improved imaging, improved therapy, improved image-guided therapy, improved serum stability, improved tumor (particularly solid tumor) penetration and/or affinity in theranostic pairing, has high practical value and promotion value in the technical field of DZ-1-Lys -DOTA conjugate.

### BRIEF DESCRIPTION OF THE DRAWINGS

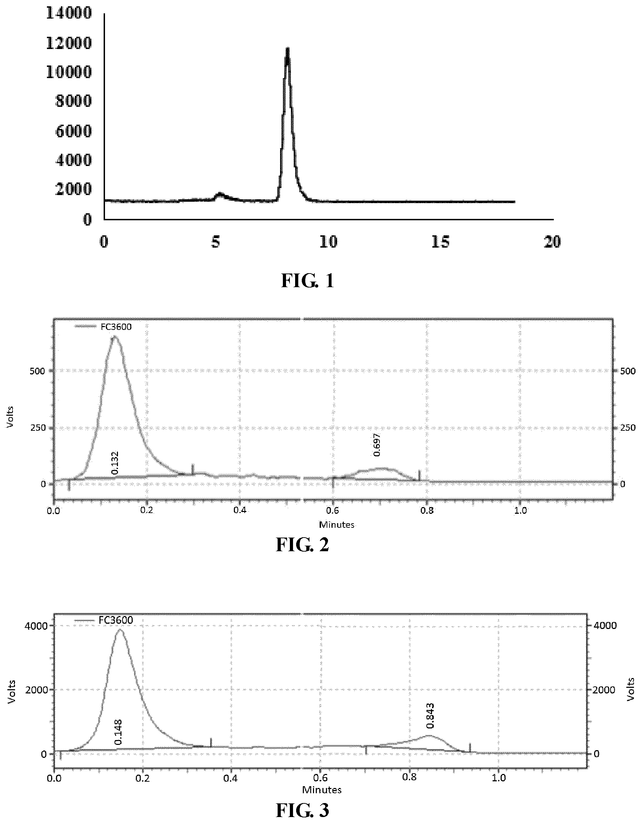

FIG. 1 is the radio-HPLC chromatogram of DZ-1-Lys-DOTA-⁶⁸Ga detected by a γ-ray detector.
FIG. 2 is the radio-HPLC chromatogram of DZ-1-Lys-DOTA-¹⁷⁷Lu detected by radioactive TLC.
FIG. 3 is the radio-HPLC chromatogram of DZ-1-Lys-DOTA-90Y detected by radio-TLC.

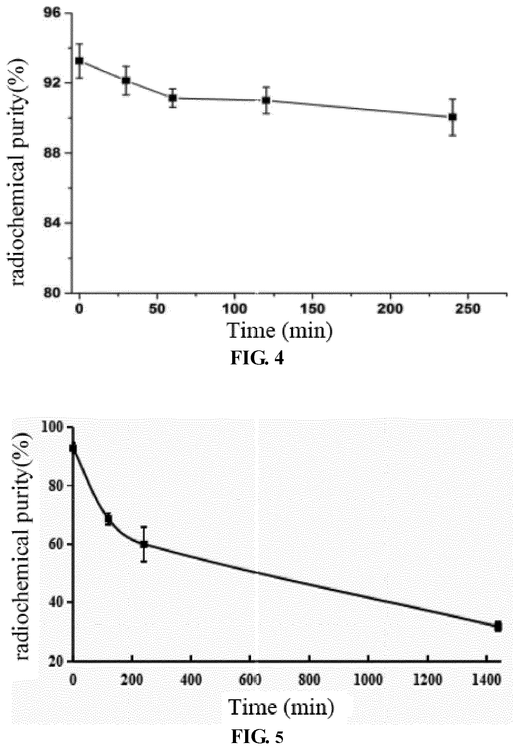

FIG. 4 shows the serum stability and radiochemical purity data of DZ-1-Lys-DOTA-68Ga at 37°C.
FIG. 5 shows the serum stability and radiochemical purity of DZ-1-Lys-DOTA-177Lu at 37°C.
FIG. 6 shows the serum stability and radiochemical purity data of DZ-1-Lys-DOTA-90Y at 37°C.
FIG. 7 shows the clearance curve of DZ-1-Lys-DOTA-⁶⁸Ga in blood of Balb/C mice.
FIG. 8 shows the clearance curve of DZ-1-Lys-DOTA-¹⁷⁷Lu in blood of Balb/C mice.
FIG. 9 shows the cytotoxicity data of DZ-1-Lys-DOTA-90Y.

### DETAILED DESCRIPTION OF THE EMBODIMENTS

In order to make the purpose, technical solutions and advantages of the present application clearer, the present invention will be further described below with reference to the examples. The embodiments of the present invention include the following examples.

This example relates to radiopharmaceutical formulations comprising conjugates of heptamethine carbocyanine dye (HMCD)-chelator radioactive complexes, and uses thereof, particularly for internal radiotherapy and/or imaging of cancer. Specifically, combinations of DZ-1-Lys-DOTA conjugates complexed with gallium-68 and lutetium-177 or yttrium-90 are provided. Some embodiments provide improved image-guided therapy through the use of a theranostic pairing of such conjugates. For therapeutic purposes, the radioactive metal is selected from lutetium-177 and yttrium-90, wherein each metal used for imaging is paired with gallium-68 for improved image-guided radiation therapy. The present invention relates to pharmaceutical compositions comprising the complexes, optionally, for use in treating cancer cells, tissues, soft tumors and solid tumors and/or metastases thereof. Advantages of embodiments may include improved treatment of cancer, tumors and metastatic tumors (including precancerous lesions), as well as improved image-guided therapy, improved serum stability, improved tumor (especially for the of solid tumors) permeability and/or the affinity of the therapeutic-diagnostic pairing.

The DZ1-Lys-DOTA radioactive metal complexes provided in the examples may be referred to herein as "coupled complexes", "dye complexes", "DOTA complexes", "dye-DOTA complexes", "HMCD-DOTA complex", "DZ1-DOTA complex", "radioactive metal complex" or simply "complex".

DZ-1-Lys-DOTA-¹⁷⁷ Lutetium complexes are particularly advantageous for smaller tumors or metastatic tumors, e.g., tumors of about 5 mm or less, 2 mm or less, 1 mm or less, or about 0.5 mm or less in width/height and length. DZ-DOTA-¹⁷⁷ Lutetium provides higher absorbed doses with high stability of the complex, which helps protect healthy tissue from being dissociated by radioactive metals.

DZ-1-Lys-DOTA-⁹⁰Y complexes can be used particularly advantageously for larger tumors, such as tumor length, height and/or width, due to their good serum stability and long penetration range, while allowing sufficiently rapid clearance from the blood circulation About 1mm or more, about 2mm or more, 5mm or more, 10mm or more, or 12mm or more.

DZ-1-Lys-DOTA-⁶⁸ Ga complexes are particularly advantageous due to their high stability in serum, e.g., compared to copper-64 complexes.

In an embodiment, pharmaceutical compositions of the invention for use in a method for image-guided radiation therapy are provided, wherein the radioactive metal for therapy is selected from lutetium-177, yttrium-90 groups, and the radioactive metal used for enhanced imaging is gallium-68. It has been found experimentally that Lu-177, Y-90 and Ga-68 all appear to be more stable in serum compared to previously known complexes, e.g. compared to the same complex of copper (Cu-64). At the same time, these complexes appear to be at least as effective in tumor-targeted therapy, tumor shrinkage or cancer cell killing.

In an embodiment, pharmaceutical compositions comprising radioactive metal complexes of DOTA chelators (see Formula I shown below) coupled via a lysine cross-linker to DZ-1, heptamethine carbocyanine dye (HMCD), and these compositions or complexes for use in radiation therapy of cancer are provided. Not part of the invention but nevertheless disclosed herein are methods of preparing the conjugates and the complexes, methods of imaging cancer cells, tissues, tumors and/or metastatic tumors , and combinations of pharmaceutical formulations for imaging and/or radiation therapy, etc. Furthermore, disclosed herein but not part of the invention are compositions for use in non-invasive imaging of complexes in vivo (particularly PET or SPECT imaging) and methods related thereto. HMCD-DOTA radioactive metal complexes can be used for radiotherapy of cancer cells and tissues (including solid tumors). These compounds may have various advantages, including improved tumor permeability.

In a first embodiment, there is provided a pharmaceutical composition comprising a first complex and a second complex of DZ1-Lys-DOTA conjugate with a radioactive metal M comprising a heptamethine carbocyanine dye (HMCD) group coupled to DOTA via a lysine crosslinker, where M in the first complex is gallium-68 and M in the second complex is lutetium-177 or yttrium-90, and the molecular formula of FI is as follows:

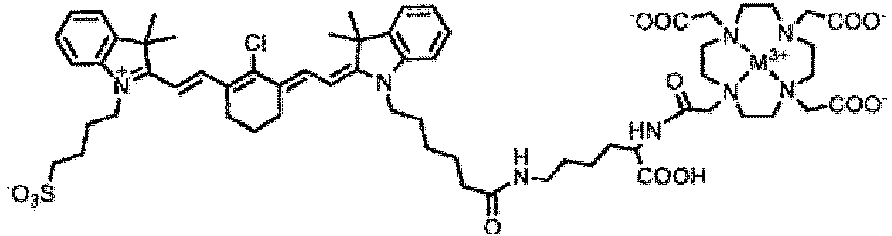

DZ-1-Lys-DOTA conjugates for the formation of radioactive metal complexes have the molecular formula of FII as shown below:

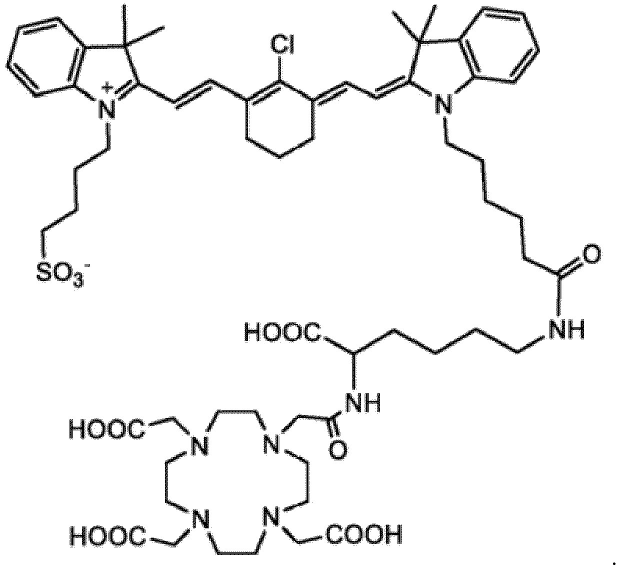

The complexes can provide one or more advantages, including that they are very stable radioactive metal complexes, which can provide an effective cancer therapy with low toxicity and good tumor penetration even in solid tumors. In particular, the complex may be suitable for therapeutic applications rather than sole imaging, and may provide one or more of the following advantages: more accurate quantification by PET or SPECT probes and/or optional near-infrared fluorescence (NIRF) analysis, higher contrast between tumor and non-cancerous tissue, higher sensitivity, better selectivity for cancer cells, especially fast stable distribution in vivo and in organs including heart, liver, lung, and kidney, rapid tumor targeting, uniform distribution in tumor tissue, absence of sustained radioactivity in one or more organs including kidney and/or liver. In addition, these complexes can advantageously be formed more easily and/or less expensively, can be readily labeled with radioactive metals, can form stable complexes with one or more radioactive metals, can have higher complexes formation rate, high radiochemical yield, good efficiency of labeling by one or more radioactive metals, good solubility in aqueous solutions, high stability in aqueous solutions, high stability at a physiological pH of about 7.4 (e.g., 7.2-7.6), high stability in vivo, low dissociation rate of radioactive metal and complex (especially low dissociation rate in vivo), good biodistribution, sufficient targeting of complex to cancer tissue, rapid cancer-specific targeting, adequate targeting for tumors and/or metastatic tumors, adequate targeting for larger tumors, low accumulation rate in organs (e.g. kidney, spleen, liver, heart), absence of or reduced radioactive exposure of tissues and organs, rapid clearance from vital or sensitive organs (e.g., from blood, liver, kidney, and other organs) following administration, adequate penetration into tissues and tumors, adequate penetration into encapsulated tissues (such as organs, including dense tissue, including tumor tissue, especially calcified tumor tissue), improved and/or prolonged residence time in the tumor.

The radiochemical yield of the isotope of a particular element refers to the yield of a radiochemical separation, expressed as a fraction or percentage of the activity originally present, also called recovery rate. In radiation chemistry, the number of species transformed by radiation per eV of absorbed energy is represented by the symbol G. That is, the G value. The radiochemical yield can be determined in a manner understood by one of ordinary skill.

The complex can penetrate the tumor more easily and more deeply. Therefore, these radiopharmaceuticals may be better suited for solid tumors, especially dense and/or larger and/or partially calcified tumors. This is particularly applicable to DZ-1-Lys-DOTA-⁹⁰Y, which is thought to provide improved treatment for larger tumors, e.g. 5-15cm³ or larger.

The complexes of this embodiment can provide blood-brain barrier (BBB) penetration and are therefore useful in the treatment of brain tumors and brain metastases.

The complexes of this embodiment may provide higher tumor aggregation rates and/or rapid blood clearance. Distribution in animals can show favorable time-dependent clearance in their organs, with specific accumulation in tumors (e.g., xenograft tumors in mice, see Examples 3 and 7).

The complexes of this embodiment may provide improved percentage and/or rate of tumor uptake, e.g., by percentage of injected dose (ID) in Formula FI (20 µg/kg) and at time points (e.g., at 4, 8, 16 and and/or 24 hours) tumor-to-blood ratio, as will be apparent to the ordinarily skilled artisan.

The complexes of this embodiment may provide increased dose selection and less accumulation and/or improved clearance from organs, including one or more of the kidneys, liver and heart. Organs such as the kidneys are often dose-limiting organs in radiation therapy. The absorbed dose can be measured, as will be apparent to one of ordinary skill in the art, for example, about 15 Gray (Gy) or less, such as 10, 5, 2, 1 Gray or less.

The complexes of this embodiment can provide reduced absorbed doses to organs and/or soft tissues (e.g., kidney, spleen, liver) and/or blood toxicity (e.g., thrombocytopenia, neutropenia).

The complex of this embodiment can provide better anticancer effect at lower radioactivity. For example, less than 50 µCi is required, such as less than 20, 5 or 2 µCi.

The complexes of this embodiment are capable of ensuring reduced toxicity at doses sufficient to treat cancer and/or significantly reduce tumor volume (e.g., at least a 10, 20, 30, 40, 50, 60, 70, 80, 90 or 100% reduction), thus reducing LD50 and/or organ toxicity, e.g., reducing toxicity in kidney, spleen, liver, bone marrow, brain, heart or lung, and/or the complex may allow avoiding or ameliorating the weight loss that often occurs during and after treatment.

The complexes of this embodiment can provide lower toxicity at doses sufficient to treat cancer and/or significantly reduce tumor volume, while improving one or more of pain scores, bone pain scores, and median survival.

The complexes of this embodiment can provide sufficient anticancer/antitumor effects at reduced total doses, e.g., less than 250 kBq/kg, e.g., less than 200, 150, 50 and 25 kBq/kg.

The complex of this embodiment can provide sufficient efficacy to treat cancer and/or significantly reduce tumor volume while avoiding one or more side effects such as pain, dizziness, nausea, effects on the digestive tract, stomach pain, constipation, diarrhea, hair loss.

The radioactive metal complex can provide therapy by the decay of the radioactive metal that deposits radiant energy in or near the target cell or tissue at a dose that kills the target cell or tissue, particularly tumor cells or tissue, or its metastatic tumors. In particular, Lu-177 and Y-90 are positron-emitting beta-emitting metals. Lu-177, Y-90 and Ga-68 can form stable complexes with the DZ-1-Lys-DOTA conjugates described herein, these complexes are stable in serum, and this therapeutic complex may provide favorable therapeutic effects and be used in the radiation therapy of cancer.

The radiation therapy performed with the pharmaceutical compositions of the invention can improve the response to primary tumors by pairing such therapy with positron emission tomography (PET) or computed tomography (CT), especially by improving the methods for detecting and/or localizing primary tumors, secondary and further tumors and/or metastases by using Ga-68 to provide improved image-guided therapy (see Examples below, especially Example 3). PET detects positron emitters and can optionally be combined with CT imaging, i.e., PET/CT. In CT, x-rays are scanned from different angles, and the different slices are arranged in a 3D form by the computer, which can be used as a map to overlay with the signal detected by PET.

The radiation therapy is performed in vivo, i.e., a radiation source is provided in a subject for cancer treatment. Internal radiation therapy can be administered systemically, so that the treatment spreads in the blood to tissues throughout the body, killing cancer cells in a targeted manner by radiation to cells in close proximity to the radioactive metal. Treatment may be administered systemically or locally/site-specifically, such as oral or intravenous injection for systemic administration, or by local injection or deposition, such as by using seeds in brachytherapy for palpable tumors, which will be apparent to those of ordinary skill. Using the stable complexes as provided, radioactive metals can be delivered to tumors and metastatic tumors in a targeted manner.

In embodiments, a complex comprising a radioactive metal selected from Lu-177 and Y-90 can be administered together with a Ga-68 complex, e.g. sequentially (administered before and/or after), or administered simultaneously with Ga-68.

In these embodiments, DZ-1-Lys-DOTA forms complexes with Ga-68 and Lu-177 or Y-90 with high thermodynamic stability and kinetic inertness, and will be stable at room temperature (at about 20 DEG C, or for example at less than 25, 30, 35, 40, 45, 50, 55, 60, 65, 70 or 75 DEG C), at near neutral pH (about 7, for example about 5 or more, about 6 or more, about 8 or less, about 7.5 or less), and at low DZ-1-Lys-DOTA concentrations, such as 0.1-100 micromolar or nanomolar, such as about 1 to about 20 micromolar, such as about 1 to about 10 micromolar, e.g., about 5-8 micromolar, allows for easy delivery of radiopharmaceutical formulations.

The complexes containing gallium-68 (⁶⁸Ga) can provide superior imaging during detection, including, for example, PET, especially one or more of better signal-to-noise ratio, stronger signal, and better resolution.

The complexes containing lutetium-177(¹⁷⁷Lu, Lu-177) can be applied to tumors of various sizes, with effects including tumor shrinkage, and can be particularly effective in destroying small tumors or metastatic tumors of a volume less than, for example, about 5 to about 0.1cm³ or smaller, for example about 5cm³ or smaller, about 4cm³or, about 3cm³or smaller, about 2cm³ or smaller, about 1cm³or smaller, about 0.5cm³or smaller, about 0.1cm³ or smaller. Tumor volumes generally apply to human patients weighing about 63.5 to 90.7 kg (e.g, about 77.1 kg) (about 140 to 200 lb (e.g., about 170 lb)), unless otherwise specified. In non-human subjects, the reduction or increase in tumor volume is dependent on body weight. Advantages may include significant reduction or removal of metastatic tumors, higher absorbed doses, and/or narrower range of tissue penetration.

The complexes containing yttrium (⁹⁰Y, Y-90) are suitable for tumors of various sizes, and their effects include tumor shrinkage. Advantages may include significant reduction or removal of metastatic tumors, higher absorbed doses, and/or narrower tissue penetration range.

Positron emission tomography (PET) may be used to provide data on the distribution of radioactive metals within the target tissue by detecting gamma photons generated from the decay of the radioactive metals. Common PET scanners with high spatial resolution can visually map the decay process of radioactive metals, thereby providing images that reflect the distribution of radioactive metal complexes in vivo after administration. Such images provide anatomical and functional information to aid medical diagnosis and assist in tracking progress and adjusting radiation therapy.

In the embodiments, cancer referred to herein includes precancerous and cancer cells or tissues, tumors and its metastatic tumors, and primary and secondary tumors. The pharmaceutical compositions described herein may be particularly useful for brain cancer and brain tumors and metastatic tumors thereof. Without wishing to be bound by theory, it is believed that DOTA binds to the radioactive metals as described, namely gallium-68 and lutetium-177 or yttrium-90. When bound via a lysine crosslinker, it allows the radioactive conjugates to penetrate through the blood-brain barrier and allows for imaging and treatment throughout the brain, including deep brain tumors, which often cannot be located effectively by other methods and may be inoperable.

In embodiments, the cancer treated with the pharmaceutical compositions of the invention includes: brain cancer, prostate cancer, lung cancer, non-small cell lung cancer (NSCLC), small cell lung cancer (SCLC), pancreatic cancer, kidney cancer, lymphoma, colorectal cancer, skin cancer, liver cancer and breast cancer, squamous cell carcinoma lung cancer, anal cancer, epithelial tumors of the head and neck, bone cancer, cervical cancer, skin cancer, melanoma, hematopoietic cell carcinoma, lymphoma and myeloma, or metastatic tumors of any of the above tumors, including but not limited to metastasis occurring in the brain, bones or other organs, brain tumors or their metastatic tumors, brain tumors and their metastatic tumors in bone, lung or other organs, bone tumors or their metastatic tumor in brain, bone, lung or other metastatic tumors , prostate tumors or their metastatic tumors in brain, bone, lung or other organs, prostate tumors and their metastatic tumors in brain, bone, lung or other organs, lung tumors and their metastatic tumors in brain, bone, lung or other organs, etc.

In embodiments, the cancer may be a central nervous system (CNS) or brain tumor, or metastatic tumor thereof, including: acoustic neuroma, astrocytoma, chordoma, CNS lymphoma, craniopharyngioma, glioma, glioblastoma medulloblastoma, meningioma, oligodendroglioma, pituitary tumor, primitive neuroectodermal tumor, schwannoma, brain stem glioma, ependymoma, juvenile pilocytic astrocytic cell tumors, optic gliomas, pineal tumors, rhabdomyomas, adult low-grade (WHO grade I or II) gliomas/capillary hemangiomas, infiltrative upper dendritic gliomas, regenerative gliomas/glioblastomas tumor, adult intracranial epididymal tumor, adult medulloblastoma, primary central nervous system lymphoma, primary spinal cord tumor, localized brain metastastic tumors, extensive brain metastatic tumors, supratentorial metastatic tumors, and metastatic spinal tumors.

In embodiments, the cancer may be non-small cell lung cancer selected from squamous cell carcinoma, adenocarcinoma (mucinous cystadenocarcinoma), large cell lung cancer, rhabdoid carcinoma, sarcoid carcinoma, carcinoid, salivary gland carcinoma, adenocarcinoma squamous cell carcinoma, papillary adenocarcinoma, giant cell carcinoma. Alternatively, the cancer may be a small cell lung cancer, including combined small cell carcinomas. Alternatively, the cancer may be a non-lung cancer, including sarcoma, lymphoma, immature teratoma, and melanoma.

The pharmaceutical compositions of the invention may comprise one or more pharmaceutically acceptable carrier and/or one or more excipients and/or one or more active substances. One or more carriers, excipients and/or active agents may be selected so as to be compatible with the other ingredients of the formulation and not be unduly deleterious to the recipient thereof. Such carriers are known in the art and can be readily selected by one of ordinary skill in the art.

In embodiments, routes of administration of the pharmaceutical compositions include, but are not limited to: oral (e.g., in a pill form), intravenous (i.e., injected into a subject's vein), interstitial injection (i.e., inserted into a body space), intraperitoneal injection, subcutaneous or intramuscular injection, and/or by brachytherapy (insertion of radioactive implants or seeds directly into the affected tissue, such as into or near the tumor site). Administration can be systemic (e.g., through the blood circulation) or topical (e.g., localized to a particular organ or part of the body). In some embodiments, the pharmaceutical compositions of the present invention comprise a pharmaceutically acceptable excipient suitable to render the pharmaceutical composition administrable via the above-described routes of administration.

In embodiments, the active ingredient may be admixed or formulated with conventional, pharmaceutically acceptable excipients or carriers. The mode of administration, vehicle, excipient or carrier should generally be substantially inert with respect to the active agent, as will be understood by those of ordinary skill in the art. Such methods, vehicles, excipients and vehicles are exemplified in, for example, Remington: The Science and Practice of Pharmaceuticals (2020), ISBN-10:0128200073 or The Handbook of Pharmaceutical Excipients 9th Edition (2020), described in ISBN-10:0857113755. The excipient must be "acceptable", that is, compatible with the other ingredients of the formulation and not harmful to the recipient.

The pharmaceutical formulations may be conveniently presented in dosage units by any method well known in the art of pharmacy. In general, such methods of preparation include presenting the formulation in a suitable form, for example, as an aqueous suspension. The dosage form may optionally contain one or more adjuvants or accessory pharmaceutical ingredients for formulation, such as admixtures, buffers, and solubilizers.

Parenteral dosage forms of pharmaceutical preparations (i.e., dosage forms that bypass the gastrointestinal tract) may include, but are not limited to, solutions ready for injection, dry products ready to be dissolved or suspended in a pharmaceutically acceptable carrier for injection, injectable suspensions and emulsions. In addition, controlled release parenteral dosage forms can also be prepared for administration to patients, including but not limited to administration dosage forms DUROS^{®}and dose dumping.

Suitable carriers that can be used to provide parenteral dosage forms of the pharmaceutical compositions of the present invention include, but are not limited to: sterile water; USP water for injection; physiological saline solution; dextrose solution; aqueous carriers such as, but not limited to, sodium chloride injection, Ringer's injection, dextrose injection, dextrose and sodium chloride injection, and lactated Ringer's injection; water-soluble carriers such as, but not limited to, ethanol, polyethylene glycol, and propylene glycol; and non-aqueous carriers such as, but not limited to, corn oil , cottonseed oil, peanut oil, sesame oil, ethyl oleate, isopropyl myristate and benzyl benzoate. Compounds that alter or modify the solubility of the pharmaceutically acceptable salts of the inventive compounds disclosed herein can also be incorporated into parenteral dosage forms of the present invention, including conventional and controlled release parenteral dosage forms.

Formulations for parenteral administration may include aqueous and non-aqueous sterile injectable solutions that also contain additional agents, such as antioxidants, buffers, bacteriostatic agents and solutes, to render the formulation compatible with the intended recipient Blood isotonic. The formulations may include aqueous and non-aqueous sterile suspensions containing suspending and thickening agents.

Injectable preparations, e.g., sterile injectable aqueous or oily suspension, can be formulated according to the known art using suitable dispersing or wetting agents and suspending agents. The sterile injectable preparation may also be a sterile injectable solution, suspension or emulsion in a nontoxic parentally acceptable diluent or solvent, for example, as a solution in 1,3-butanediol. Acceptable vehicles and solvents that may be employed include water, Ringer's solution, U.S.P. and isotonic sodium chloride solution. In addition, sterile fixed oils are conventionally employed as a solvent or suspending medium. For this purpose, any bland fixed oil can be employed including synthetic monoglyceride or diglycerides. In addition, fatty acids such as oleic acid are used in the preparation of injections.

Forms suitable for oral administration include tablets, troches, capsules, elixirs, suspensions, medicated syrups, wafers, or the like, prepared by art-recognized procedures. The amount of active compound in such therapeutically useful compositions or formulations is such that a suitable dosage will be obtained. Syrup formulations generally comprise suspensions or solutions of the compound or salt in a liquid carrier such as ethanol, glycerol or water, with the addition of a flavoring or coloring agent.

Solid dosage forms for oral administration may include capsules, tablets, pills, powders and granules. In such solid dosage forms, the active compound is mixed with at least one inert substance, and/or a) fillers or fillers such as starch, lactose, sucrose, glucose, mannitol and silicic acid, b) binders such as carboxylate methylcellulose, alginate, gelatin, polyvinylpyrrolidone, sucrose and acacia, c) humectants such as glycerol, d) disintegrants such as agar, calcium carbonate, potato or tapioca starch, alginic acid, certain silicates and sodium carbonates, e) solution retarders such as paraffin waxes, f) absorption enhancers such as quaternary ammonium compounds, g) wetting agents such as cetyl alcohol and glyceryl monostearate, h) absorbents, such as kaolin and bentonite, and i) lubricants such as talc, calcium stearate, magnesium stearate, solid polyethylene glycols, sodium lauryl sulfate, and mixtures thereof. In the case of taking capsules, tablets and pills, the dosage form may also contain buffering agents.

In similar types of solid compositions, excipients such as lactose or milk sugar and high molecular weight polyethylene glycols may be employed as fillers for soft and hard gelatin capsules. Solid dosage forms of tablets, formulations, capsules, pills and granules can be prepared with coatings and shells such as enteric coatings and other coatings well known in the pharmaceutical formulation art. They may optionally contain opacifying agents and may also be of such a composition that they release the active ingredient selectively in a delayed manner only or preferentially in a certain part of the intestinal tract. Examples of embedded components that can be used include polymeric substances and waxes. Similar types of solid compositions can also employ excipients such as lactose or milk sugar and high molecular weight polyethylene glycols as fillers in soft- and hard-fill gelatin capsules.

The complexes can be in microencapsulated form with one or more excipients as described above. Solid dosage forms of tablets, formulations, capsules, pills and granules can be prepared with coatings and shells such as enteric coatings, controlled release coatings and other coatings well known in the pharmaceutical formulation art. In such solid dosage forms, the active compound may be mixed with at least one inert diluent such as sucrose, lactose and starch. Such dosage forms may also contain, as is common practice, additional substances other than inert diluents, such as tableting lubricants and other tableting aids, such as magnesium stearate and microcrystalline cellulose. In the case of preparing capsules, tablets and pills, the dosage form may also contain buffering agents. They may optionally contain opacifying agents and may also be of such a composition that they release the active ingredient selectively in a delayed manner only or preferentially in a certain part of the intestinal tract. Examples of embedded components that can be used include polymeric substances and waxes.

The effective amount, toxicity and therapeutic effect of the complexes can be determined by standard pharmaceutical procedures in cell cultures or experimental animals, e.g., for the determination of LD50 (for 50% of the population) lethal dose) and ED50 (the therapeutically effective dose for 50% of the population). The dosage may vary depending upon the dosage form employed and the route of administration employed. The dose ratio between toxic and therapeutic effects is the therapeutic index and can be expressed as the ratio LD50/ED50. The compositions may exhibit large therapeutic indices. A therapeutically effective dose can be estimated initially by cell culture assays. In addition, dosages can be formulated in animal models to achieve a circulating plasma concentration range that includes the IC50 (i.e., the concentration of a compound that achieves half-maximal inhibition of symptoms) as determined in cell culture or in an appropriate animal model. For example, plasma concentrations can be determined by high performance liquid chromatography. The effects of any particular dose can be monitored by appropriate bioassays. The dose can be determined by the physician and adjusted as necessary to suit the observed therapeutic effect.

The dosage of the pharmaceutical formulations described herein can be determined by a physician and adjusted as needed to accommodate the therapeutic effect observed. With regard to the duration and frequency of treatment, the skilled clinician will typically monitor the subject to determine the effect of the treatment and to determine whether to increase or decrease the dose, increase or decrease the frequency of administration, discontinue treatment, resume treatment, or make other changes to the regimen. The dosage schedule/regimen may vary, e.g., weekly, daily, or at specific predetermined intervals, depending on a number of clinical factors, such as the subject's sensitivity to each active compound.

An effective dose of the composition comprising the radioactive metal complex can be administered to the patient once. Alternatively, the effective dose of the composition comprising the radioactive metal complex can be repeatedly administered to the patient. The radioactive metal complexes can be administered over a period of time, e.g., within a period of 5 to 60 minutes, e.g., about 30 minutes. If necessary, the administration may be repeated periodically, e.g., hourly, daily, biweekly or weekly, e.g., at appropriate time intervals, e.g., about 6, 12, 24, 48 or 72 hours. In some cases, the frequency of treatment can be reduced after the initial treatment regimen. For example, after the initial administration, repeated administrations may be performed weekly, monthly, six months, or a year or longer.

The amount of the first complex and the second complex in the pharmaceutical composition depends on weight, mole or volume. The pharmaceutical composition may comprise at least 0.0001%, 0.1%, 0.5%, 1%, 2%, 3%, 4%, 5% or 10% of the first complex and the second complex. In some embodiments, the pharmaceutical composition comprises 0.01%-99% of the first complex and the second complex, e.g., 0.05%-90%, 0.1%-85%, 0.5%-80%, 1%-75%, 2%-70%, 3%-65%, 4%-60% or 5%-50% of the first complex and the second complex.

The dosage of the composition of this embodiment can be determined by the physician and adjusted as needed to suit the therapeutic effect observed. With regard to the duration and frequency of treatment, the skilled clinician will typically monitor the subject to determine the therapeutic effect of the treatment and to determine whether to increase or decrease the dose, increase or decrease the frequency of administration, discontinue treatment, resume treatment, or other changes to the treatment plan. The dosing schedule can vary from weekly to daily depending on several clinical factors, such as the subject's sensitivity to the compounds of the invention.

In addition to treating cancers (precancerous or cancer cells and tumors) in a subject in need of treatment, such cancers need to be identified, imaged and/or localized. This can include providing a radioactive metal complex; administering the complex to a subject; and optionally performing imaging, such as PET imaging, to detect the released emitter. This allows for visual tracking of tumor growth and/or shrinkage, such as confirmation or individualized optimization of doses, and/or location of tumors and/or metastatic tumors. Imaging can be performed, for example, within about 6 to 48 hours after administration. Imaging can be compared to normal tissue/cells to determine different uptake rates in cancer/tumor tissue versus normal tissue.

In situ pharmacokinetic and pharmacodynamic analysis can be performed in tumor or normal cells or tissues. This may comprise providing the complex; contacting the complex with cancer cells, tumor or normal cells or tissue; imaging the cancer cells, tumor or normal cells or tissue, followed by pharmacokinetic and/or pharmacodynamic analysis, For example, a signal (including radioactive and/or near-infrared fluorescence and changes thereof) can be determined as a function of time at one or more time points over a period of time, e.g., before and after administration, and at one or more time points after administration.

The concentration of the complexes employed in the pharmaceutical composition and/or the amount administered to the patient or subject can vary depending on a variety of factors including, for example, the particular complexes and/or pharmaceutically acceptable carrier used, the specific disease being treated, the extent of the disease, the age and weight of the patient, etc. Typically, complexes can be used in such pharmaceutical compositions, and the compositions can provide a patient with an initial lower level of radiation dose, which can be increased until the desired therapeutic effect is achieved. In general, the complexes can be used in pharmaceutical compositions comprising an aqueous carrier to provide absolute radioactivity concentrations ranging from about 4 mbq/ml (about 0.1 mci/ml) or less to about 370 mbq/ml (about 10 mci/ml), and all combinations and subcombinations of ranges therein. The concentration of the complexes in the pharmaceutical composition may be from about 37 MBq/ml (about 1 mCi/ml) to about 370 MBq/ml (about 10 mCi/ml). Additionally, the compositions can be administered to patients in doses ranging from about 1 KSv (about 1 x 1x10⁵Rem) to about 74KSv (about 7.4MRem), and all combinations and subcombinations of ranges therein. The composition can be administered to a patient to provide about 7.4 KSv (about 7.4x10⁵Rem) to a radiation dose of about 74KSv (about 7.4MRem). These amounts are referred to herein as effective or therapeutically effective amounts. The pharmaceutically acceptable carrier should also contain a thickening agent.

Thickeners refer to any of a variety of generally hydrophilic materials that, when incorporated into the present compositions, can act as viscosity modifiers, emulsifiers and/or solubilizers, suspending agents and/or enhancers. Thickening agents suitable for use in radiopharmaceutical compositions include, for example, gelatin, starch, gums, pectin, casein and algins, including carrageenan, alginate and agar, hemisynthetic cellulose derivatives, poly vinyl alcohol and carboxyvinylates, bentonites, silicates and colloidal silica. Other thickeners will be apparent to those of ordinary skill.

The concentration of the thickening agent can range from about 0.1 milligrams (mg) to about 500 milligrams (mg) per milliliter of the pharmaceutical composition. The concentration of the thickening agent may be about 1 to about 400 mg/ml, such as about 5 to about 300 mg/ml, such as about 10 to about 200 mg/ml, such as about 20 to about 100 mg/ml, or for example from about 25 to about 50 mg/ml. Compositions that can be prepared from the complexes, pharmaceutically acceptable carrier and optional thickening agent include, for example, suspensions, emulsions and dispersions. The complexes can be formulated and administered to a patient as a suspension.

Suspension may refer to a mixture, dispersion or emulsion of finely dispersed colloidal particles in a liquid. For example, suspensions can be obtained by combining the complexes with an inert solid support material. Particulate support materials that may be suitable for use as inert solid supports in the compositions of the present invention include, for example, materials derived from carbon, including those forms of carbon commonly referred to as carbon black (soot) and/or activated carbon, as well as fine powder oxides, diatomaceous earth, and diatomaceous earth. The support material comprises carbon black or activated carbon. The size of the particles of the particulate carrier material can vary and depend upon, as used, the particular carrier material, compound, thickener, etc., and can include particles ranging in size from, for example, from about 0.1 micrometer (mm) to about 50 mm. For example, the particle size may be from about 0.5 to about 25 mm, e.g., from about 1 mm to about 10 mm, e.g., from about 2 mm to about 5 mm.

Delivery of the therapeutically effective activity of the complexes can be obtained by administering a pharmaceutical composition comprising the therapeutically effective activity or dose of the complexes, i.e., at a concentration sufficient to elicit the desired therapeutic or imaging effect. An effective therapeutic activity can be an activity effective to treat cancer, such as inhibiting or slowing the growth of cancer or precancerous tissue, or reducing the survival rate of cancer or precancerous cells. The therapeutically effective dose varies from patient to patient and depends on the patient's condition and route of administration and varies according to subject's weight, sex, age and medical history. Other factors that affect the effective dose may include, but are not limited to, the severity of the patient's condition, the disease or disorder being treated, the stability of the complexes, and, if applicable, any additional antineoplastic therapeutics administered with the complexes. Methods for determining efficacy and dosage are known to those of ordinary skill.

### Example 1 Synthesis of coupled DZ-1-Lys-DOTA radioactive metal complex

The synthesis of DZ-1 dye and its derivatives has been described previously, e.g. in US 10307489, and can be performed as described in detail in Example 1a below. The conjugate can then be complexed with the radioactive metal as described in Example 1b. The following chemicals and reagents can be used. DOTA, also known as 1,4,7,10-tetraazacyclododecane-1,4,7,10-tetraacetic acid, DOTA tri (tert-butyl ester),also known as 1,4,7,10-tetraazacyclododecane-1,4,7-tri-tert-butylacetic acid-10-acetic acid, available from Macrocyclics (Arlington Heights, IL). DOTA and all other chemicals mentioned herein, such as in the reaction schemes as shown, can also be purchased from various standard sources, such as VWR International (Radnor, PA) or Thermo Fisher Scientific (Waltham, PA). MA), as will be apparent to those of ordinary skill. Deionized ultrapure water (18.2 MΩ) can be used to prepare the solution, which is available from the Milli-Q direct ultrapure water system from Millipore Corporation (Billerica, MA, USA). Analytical reversed-phase (RP) high performance liquid chromatography (HPLC) can be performed on an Agilent system, e.g., using a 1260 infinite diode array detector, e.g., using an Apollo C18 RP column (5 µm, 150×4.6 mm). For example, the mobile phase can be changed from 60% solvent A (e.g., 0.1% trifluoroacetic acid in 80% water) and 40% solvent B (e.g., 0.1% trifluoroacetic acid in 80% acetonitrile in water) in, e.g., about 30 minutes was changed to 100% solvent B at a flow rate of about 1 ml/min for example at about 254 nm and about 780 nm over a period of time. Electrospray ionization mass spectrometry (ESI-MS) analysis can be performed on synthetic compounds, e.g., on a Thermo LTQ Orbitrap Elite mass spectrometer system.

### Example 1a Synthesis of DZ-1-Lys-DOTA conjugate

Scheme 1, shown below, illustrates the formation of the DZ-1-Lys-DOTA conjugate, the first step of the coupling of compound 4, also referred to herein as "DZ-1". Other synthetic routes are apparent to those of ordinary skill in the art. DZ-1 can then be coupled to a lysine crosslinker (shown in Scheme 2 below), which is further coupled to DOTA (shown in Schemes 3 and 4 below (or Scheme 4 of alternative 5)).

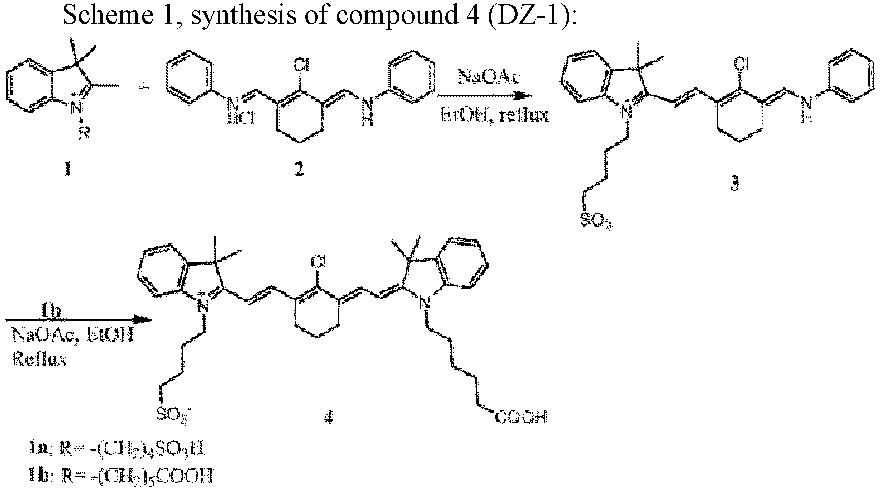

Synthesis of Compound 1a (compare Scheme 1 above): A mixture of 2,3,3-trimethylindole (5 g, 31.4 mmol) and 1,4-butane sulfonate lactone (5.1 g, 37.7 mmol) can be heated with stirring at 120°C for 5 hours under argon. The resulting reaction mixture is cooled to room temperature (e.g., about 20°C), and the solid can be dissolved in a sufficient volume of solvent, e.g., and an organic solvent, e.g., about 50 mL of methanol. Add about 200 mL of diethyl ether to methanol solution for precipitation, and collect the precipitate, wash with sufficient volume and times of ethyl acetate (e.g., 15 mL, three times), and dry, for example, under vacuum, to obtain the desired solution. Desired product 6.8 g (73% yield) as a white solid.

Synthesis of Compound lb (compare with Scheme 1 above): To 6-bromohexanoic acid (2.5g, 13.0 mmol) was added 2,3,3-trimethylindoline (2.5g, 15.7 mmol). The reaction mixture was heated with stirring at a sufficiently elevated temperature, e.g., at about 110°C for 8 hours under a protective gas (e.g., argon). The resulting dark red solid could be dissolved in 50 ml of methanol. Then 150 ml of ether were added. The precipitate was filtered and washed a sufficient number of times with diethyl ether (e.g., 15 ml, three times) and then with acetone (e.g., 15 ml, three times). The resulting product was a white solid (2.7 g, 58%).

Synthesis of compound 3 (compare with Scheme 1 above): To a mixture of 1a (2 g, 6.78 mmol) and compound 2 (3 g, 8.36 mmol) in ethanol (100 ml) was added sodium acetate (0.28 g, 3.39 mmol), the resulting mixture was heated at a sufficient temperature and for a period of time, for example at about 60°C for about 18 hours. The precipitate can be filtered and washed thoroughly with cold ethanol (e.g., 20 ml, three times). The product was dried under vacuum to give the desired product 3 as a dark blue solid (2.1 g, 58% yield). Mass spectrum (ESI) 525.19 [M+H]⁺.

Synthesis of compound 4, a heptamethine carbocyanine dye also referred to herein as DZ-1 (compare with Scheme 1 above): To compound 1b (0.67 g, 1.9 mmol) and compound 3 (1.0 g, 1.9 mmol) in ethanol (20 ml), sodium acetate (156 mg, 1.9 mmol) can be added and the resulting solution can be heated to reflux for a sufficient time, e.g. 3 hours. The mixture can then be precipitated, e.g., 100 ml of ice water is poured into the drug formulation combination. The solid can be filtered and crystallized from methanol-water to give the desired dark green solid 4 (0.99 g, 74% yield). Mass spectrum (ESI) 705.31 [M+H]⁺.

Scheme 2 below shows the synthetic coupling of a dye coupled to a suitable linker group, here DZ-1-Lys:

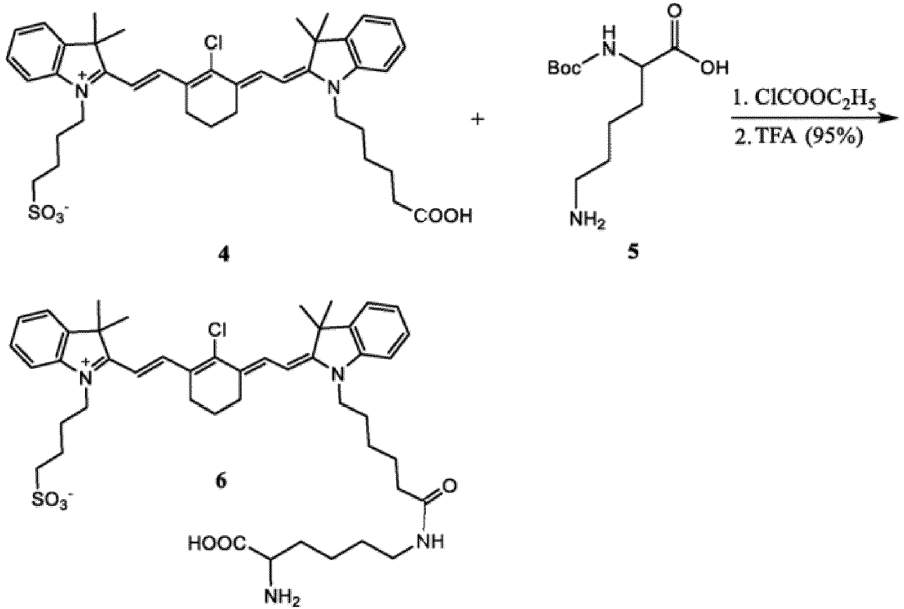

Synthesis of DZ-1-Lys (compare Scheme 2 above): DZ-14 (200 mg, 0.28 mmol) was dissolved in 5 ml of dry CH₂Cl₂. Ethyl chloroformate (ClCOOC₂H₅) (46 mg, 0.42 mmol)) and triethylamine (57 mg, 0.57 mmol) could be added. The mixture could be stirred for a sufficient time (e.g., about 2 hours), then a sufficient volume (e.g., 2 ml DMF) of N-α-Boc-Lys 5 (70 mg, 0.28 mmol) can be added and stirred for a sufficient time (e.g., room temperature (RT) about 2 hours). The crude material can be precipitated in cold diethyl ether (40 ml). Centrifugation for sufficient time and rotation speed (e.g., about 5 minutes at about 3500 rpm) enables recovery of purifiable particles, e.g., by C18-reversed silica gel chromatography eluting with acetonitrile in aqueous ammonium bicarbonate (20 mM) to give Desired product DZ-1-(N-α-Boc)-Lys as a dark green solid 109 mg (42%). DZ-1-(N-α-Boc)-Lys can be dissolved in TFA (95%) 5ml, and the mixture can be stirred well, e.g., at ambient temperature for about 3 hours, e.g., at room temperature (about 20°C) under stirring. Sufficient diethyl ether can be added, e.g., 40ml. Decantation of the ether can be achieved by centrifuging the suspension for a sufficient time to achieve separation. The product can be dried, e.g., placed under high vacuum for a sufficient time, e.g., overnight. The resulting product 6 was used in the following steps without further purification.

Schemes 3 and 4 (or alternative 5 show the synthesis of activated chelator compounds (here: DOTA sulfoNHS, compare with Scheme 3), which can then be coupled with DZ-1-Lys compounds (compare Scheme 4 for the first method, or alternative 5 for the second method).

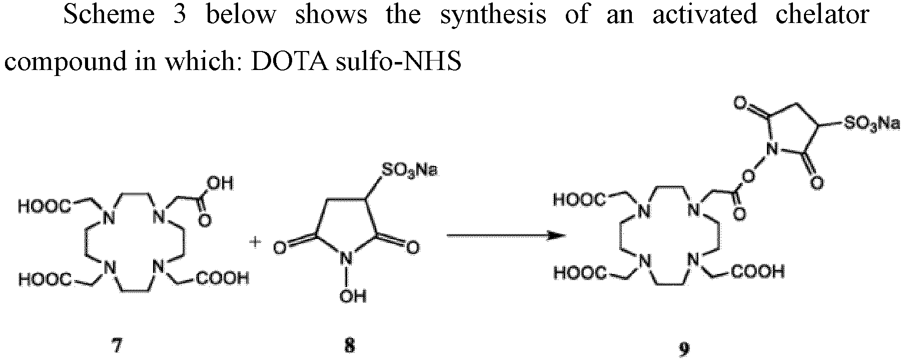

Synthesis of DOTA Sulfo-NHS (compare with Scheme 3 above): DOTA 7 can be prepared at a suitable pH, for example a pH of about 5.5, for a suitable time, for example about 30 minutes (4°C), with a suitable molar ratio (e.g. DOTA:EDC:Sulfo-NHS) in a molar ratio of about 10:5:4, activated with EDC. DOTA (24.2 mg, 48 µmol) can be dissolved in an appropriate amount of water and solvent, for example, about 500 µL of water, and about 4.6 mg of EDC (24 µmol) in an appropriate volume of water (for example, about 130 µL of water), and mixed. After cooling (e.g. in an ice bath) to 4°C, sulfo-NHS (4.2 mg, 19.2 µmol) was added to the stirred mixture and further 0.1 N NaOH was added to adjust the pH appropriately, e.g. a final pH of about 5.5. The reaction can be continued for an appropriate time, for example, at about 4°C for about 40 minutes.

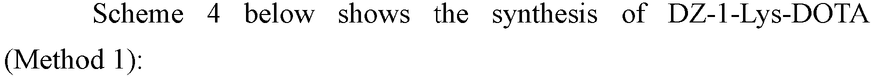

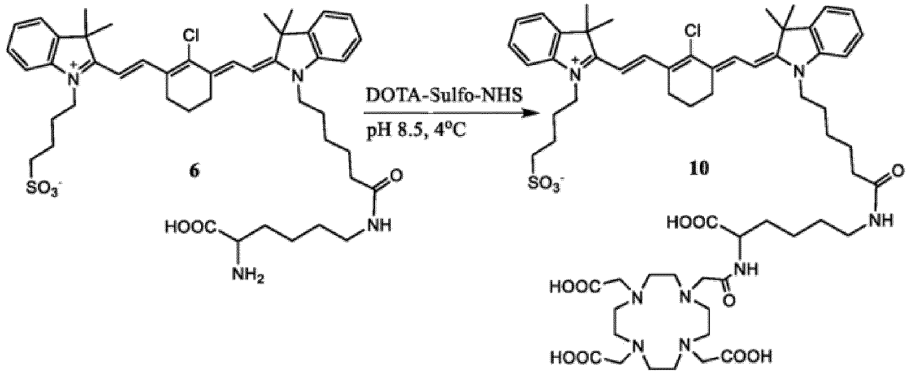

Synthesis of DZ-1-Lys-DOTA 10 (compare with Scheme 4 above): DZ-1-Lys 6 (10 mg, 12 µmol) in 200 µL of 50% acetonitrile in water was added to the DOTA sulfo-NHS 9 reaction mixture and the pH was adjusted to an appropriate alkaline pH, e.g., to about 8.5 with 0.1 N NaOH. The reaction can be incubated at a sufficiently low temperature, e.g., about 4°C, for a suitable time, e.g., overnight. The DZ-1-DOTA conjugate can be purified by a suitable separation method, e.g., by HPLC, e.g., using an Apollo C18 semi-preparative column (e.g., 5 µm, 250×10 mm). Two mobile phases were available for HPLC: solvent A (0.1% TFA in water) and solvent B (0.1% TFA in 80% acetonitrile in water). The mobile phase gradient can be varied from 40% B to 100% B in about 30 minutes at a flow rate of about 3 ml/min, monitored at dual wavelengths of 254 and 780 nm. This product can be collected and then lyophilized: 4 mg (28%). Mass spectrum (ESI) 1219.585[M+H]⁺.

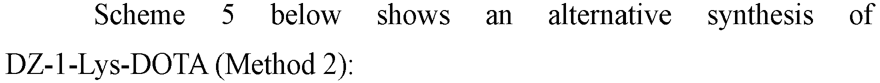

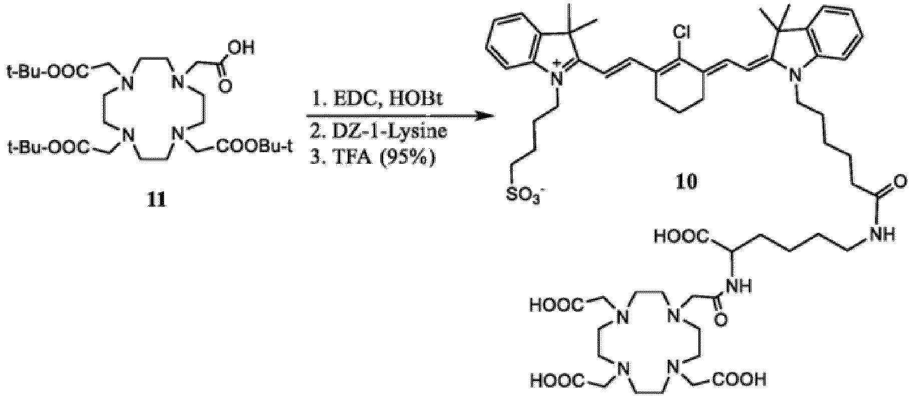

Alternatively, the synthesis of DZ-1-Lys-DOTA 10 can be carried out as follows (Method 2): a mixture of DOTA-tri (tert-butyl ester) 11 (50mg, 0.087mmol) N-ethyl-N'-(3-diethyl ester) carbodiimide hydrochloride (25mg, 0.13mmol) and 1-hydroxy-7-azabenzotriazole (15mg, 0.11mmol) may be dissolved in 3 ml of DMF. The mixture can be stirred for 40 minutes, then DZ-1-Lys 6 (73 mg, 0.087mmol) was added and stirred for an appropriate time, e.g., at room temperature for about 5 hours. The product can be precipitated, e.g., in cold ether (e.g., about 40 ml). Recovery can be performed by centrifugation at sufficient rotational speed for a sufficient time, e.g., centrifugation at 3500 RPM for 5 minutes. The particles can be dissolved in an appropriate volume of an appropriate solvent, e.g., in about 2 ml of TFA (95%), and stirred for a sufficient time, e.g., about 3 hours. The crude product can be precipitated e.g., in cold ether (e.g. about 40ml) and purified e.g. by C18-reverse semi-preparation agent to give DZ-1-Lys-DOTA 10 as a dark green solid 33mg (31%).

### Example 1b - Labeling of DZ-1-Lys-DOTA conjugate to form DZ-1-Lys-DOTA-M (here: Lu-177)

The DZ-1-Lys-DOTA conjugate can be labeled, i.e., chelated, with the radioactive metal ("M") in any convenient manner apparent to those of ordinary skill; for example, the radioactive metal M can be introduced in an ionic form, the cationic radioactive metal (M For example, selected from but not limited to:¹⁷⁷Lu³⁺, ⁹⁰Y³⁺, ⁶⁸Gd³⁺) with anionic reagents such as chloride (i.e., Mⁿ⁺Clₙ) paired, as shown in Scheme 6 below, to form such as but not limited to: ¹⁷⁷LuCl₃, ⁹⁰YCl₃, ⁶⁸GaCl₃.

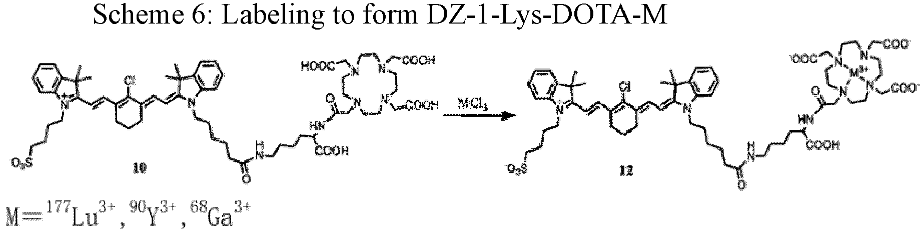

For radiolabeling, an appropriate radioactive amount of radioactive metal can be added to the DZ1-Lys-DOTA conjugate, for example at about 2 to about 200 MBq/µg (radioactive metal: conjugate), about 4 to about 100 MBq/µg or about 8 to about 50 MBq/µg, e.g., about 20 MBq/µg. For example, 100MBq (2.7mCi) of ¹⁷⁷LuCl₃ Add to 5 µg of DZ-1-Lys-DOTA, can be added to 0.1N ammonium acetate (pH 5.5) buffer, and the mixture was incubated at an appropriate temperature for sufficient time to achieve complete labeling, for example at about 20°C to about at 60°C or about 30°C to about 50°C, e.g., about 40°C for about 10 to about 60 minutes or about 20 to about 40 minutes, e.g., about 30 minutes.

For example, radiolabeling can be done by adding 100 MBq (2.7mCi) to 5 µg DZ-1-Lys-DOTA in 0.1 N ammonium acetate (pH 5.5) buffer¹⁷⁷LuCl₃ and incubating at about 40°C for about 30 minutes. DZ-1-Lys-DOTA-¹⁷⁷LuCl₃The complex can be purified by reverse phase HPLC using an Apollo C18 RP column (5µ, 250×10 mm). The column eluate can be monitored by UV absorbance at 254 nm and a NaI crystal detector. The mobile phase was changed from 40% solvent A (0.1% trifluoroacetic acid in 80% water) and 60% solvent B (0.1% trifluoroacetic acid in 80% acetonitrile in water) at a flow rate of 3 ml/min in 30 minutes 100% Solvent B. DZ-1-Lys-DOTA-M from HPLC³⁺ Pure fractions of the complex can be dried and concentrated by gently blowing through a positive flow of nitrogen. The residue left in the tube after concentration can be reconstituted in a suitable buffer, e.g., 1XPBS buffer (1.0ml), depending on further testing or dosing.

⁹⁰Y³+and⁶⁸Ga³+The DZ-1-Lys-DOTA complex can be formed and labled by using a similar protocol, e.g. by using ⁹⁰YCl₃or⁶⁸GaCl₃, techniques that are apparent to those of ordinary skill.

### Example 2: Targeted radiotherapy, tumor weight loss, and weight preservation in mice

To evaluate¹⁷⁷ the therapeutic efficacy, efficacy and toxicity of Lu-DZ-1-Lys-DOTA, human brain cancer cells (e.g., neuroblastoma, astrocytoma or glioblastoma cells, published e.g., from ATCC) can be subcutaneously inoculated in nude mice. Subcutaneous tumors of approximately ~100 mm³ in size in nude mice were randomly divided into groups (e.g., n=5 mice per group). For treatment groups, tumor-bearing mice will be injected intravenously (e.g., through the tail vein), e.g., about 0.2ml of ¹⁷⁷Lu-DZ-1-Lys-DOTA (5.55GBq/kg) Lys. Treatment may be given on a regular basis, such as daily or weekly, for several days or weeks, such as weekly for 4 weeks. Under the same treatment plan, the control group can be injected with a vehicle (such as an equal volume of normal saline, such as 0.2 ml) via the tail vein. After treatment, the mice can be housed for an additional period of time, such as 12 weeks. The endpoint of the study will be the difference in tumor growth between the treatment and control groups. Body weight can be monitored throughout the procedure.

Alternatively, ¹⁷⁷The therapeutic efficacy, efficacy, and toxicity of Lu-DZ-1-Lys-DOTA can basically be determined using the nude mouse experiments described above, except that the inoculation of human brain cancer cells is performed intracranially, and the endpoint of the study is differences in animal mortality between the treatment group and the control groups. Body weight can be monitored throughout the procedure.

¹⁷⁷Tumor-specific targeting of Lu-DZ-1-Lys-DOTA can be determined by one or more methods, such as 1) near-infrared fluorescence tumor imaging to detect accumulation of the DZ-1 moiety in tumors, and 2)¹⁷⁷SPECT/CT nuclear imaging of Lu in the same tumor. Tumor size can be measured periodically, such as once or twice a day or week, using a suitable method, such as using a caliper, such as a digital caliper, and tumor volume can be calculated using the following formula: volume = 1/2 (length × width × width) . To monitor potential toxicity, body weight can be measured. When the tumor volume exceeds 1,500mm³ or reduction in body weight >20%, mice can be euthanized. The complexes of this example may advantageously exhibit reduced tumor weight, demonstrate efficacy, preserve body weight, and demonstrate lack of toxicity.

⁹⁰Y-DZ-1-Lys-DOTA can be used in the above scheme except ¹⁷⁷Lu-DZ-1-Lys-DOTA.

### Example 3: PET/MicroPET imaging

In this example, PET (or micro-PET) can be combined with¹⁷⁷Lu- or⁹⁰Y-complexes are performed together to provide image-guided therapy. Without being bound by theory, we believe that with¹⁷⁷Lu, ⁹⁰Y, or a combination of the two, provides a well-matched true therapeutic pair that enables therapeutic radioactive metals to destroy tumors under the guidance of imaging radioactive metals, possibly due to their pharmacokinetics, stability, and efficacy, including due to similar binding, tumor uptake and organ clearance.

Micro-PET imaging is PET imaging performed on small animals, as will be apparent to those of ordinary skill. DZ-1-Lys-DOTA labeled with Ga-68 can be administered intravenously, e.g., in groups of five mice, by injecting, e.g., about 300-500 µCi of the complex. Transaxial microPET images can be collected at suitable time intervals, for example at 1, 2 and 3 hours (pi) time points after probe injection. Normalized uptake value (SUV) analysis can be performed on tumor xenografts and skeletal muscle of individual mice, as defined by CT scans, and tumor-to-muscle ratios can be calculated for each group at these time points. Blood clearance and organ distribution: After anesthesia (e.g., with isoflurane 2-3%), a group of mice (e.g., 5) can be injected with radiolabeled⁶⁸The Ga-DZ-1-Lys-DOTA complex (approximately 5 µCi) is injected, for example, via the tail vein. Retro-orbital blood samples (25 µl) can be collected at different time points, e.g., 5, 15, 30, 60 and 180 min after injection, and radioactivity of all samples can be assessed in a gamma counter (e.g.: 1480Wizard, Perkin-Environment) Counts were counted, and normalized plotted against injection time, followed by nonlinear regression analysis to obtain half-life time in blood (e.g., by Prism^{™} Software, such as Prism version 9, available from GraphPad, San Diego, CA^{™} publicly available). Mice can be sacrificed immediately after the last blood drawing. Tumors and organs such as heart, liver, lung, kidney, small intestine, stomach, bone, muscle, spleen and skin can be collected and counted for radioactivity in a gamma counter. Organ distribution data of the DZ-1-Lys-DOTA complex in mice can be obtained in duplicate at multiple time points (e.g., three time points, e.g., 1 hour, 2 hours, and 3 hours). Image registration and analysis can be performed as follows: PET/CT images can be processed according to standard protocols that will be apparent to the ordinarily skilled artisan, e.g. using standard software according to the manufacturer's guidelines, e.g. using Siemens Healthineers^{™}, Erlangen, Germany^{™} ASIPro^{™} software. Pixel-level normalized uptake values (SUVs) for PET can be calculated as pixel-level activity divided by the product of injected dose and body weight. Tumor targets can be delineated at 40% of the SUV maximum, anatomically superimposed with CT images by software image registration. The complexes of this example may advantageously display a specific profile characterized by high uptake in tumors. The distribution ratio between tumor and skeletal muscle can be, for example, about 8:1 after 24 hours, and can be, for example, after administration ⁶⁸Ga-DZ-1-Lys-DOTA was higher than 20:1 after 48 hours.

### Example 4: Stability test of Lu-177, Y-90 and Ga-68 complexes in serum

The serum stability of the complexes described herein can be determined as follows. A suitable amount, e.g., 50 microcoul, of a complex described herein, e.g., a complex of DZ-1-Lys-DOTA radioactive metal complex with Lu-177, Y-90, or Ga-68, can be added to an appropriate amount, e.g., Lys in 100 µl of fetal bovine serum (Invitrogen, Grand Island, NY). After incubation at 37°C at appropriate time intervals (e.g., 1, 3, and 6 hours), the mixture was aliquoted and filtered, e.g., through a 0.2 µM micro-spin filter. The resulting filtrate can be analyzed by suitable separation and detection methods, e.g., reversed-phase HPLC with a Bioscan flowmeter digital radio HPLC detector. The original peaks represent unfractionated stable complexes (here DZ-1-Lys-DOTA complexes with Lu-177, Y-90 or Ga-68, compare e.g. compound 12 shown above). Detection of any newly formed gamma peaks, in addition to the original gamma peaks, identifies degradation products and thus the lack of stability of the complexes in serum. Compared with Ga-68, Lu-177 or Y-90 complexes, analysis of the corresponding complexes with Cu-64 showed that the Cu-64 complex lacked stability, especially DZ-1-Lys-DOTA-Lu- 177, DZ-1-Lys-DOTA-Y-90 and DZ-1-Lys-DOTA-Ga-68. Therefore, Lu-177, Y-90 and Ga-68 complexes provide excellent serum stability.

### Example 5: Image-guided therapy of prostate cancer

4-6 week old male nude mice can be inoculated subcutaneously with human prostate cancer cells such as C4-2B (also known as ATCC^{®} CRL-3315^{™}) can be publicly available from the American Type Culture Collection (ATCC) or ARCaPM, Manassas, VA, and ARCaPM cells (catalog number: 3422, human prostate cancer cells established from parental mixed ARCaP cell populations with a high propensity for bone metastasis in mice) can be publicly available from Novicure ^{™}, Birmingham, AL. The histopathology of bone tumors was predominantly osteoblastic, which reproduced bone metastasis from prostate cancer. ARCaPM cells (spindle-shaped mesenchymal morphology) were obtained by single-cell cloning of parental ARCaP cells. ARCaPM are highly aggressive prostate cancer metastatic cells. In one study, the incidence of bone metastases was determined to be 100% (9/9) after intracardiac injection of ARCaPM cells, with incubation periods of 71 and 61 days, respectively. ARCaPM cells can be cultured and grown by using the MCaP medium provided by company Novicure^{™}. ARCaPM cells can be used to study prostate cancer bone metastasis and the role of EMT in tumor metastasis. MCaP medium was prepared with Dulbeccos' modified eagle and F12K medium, containing essential and non-essential amino acids, vitamins, organic and inorganic compounds, hormones, growth factors and trace elements, supplemented with several factors crucial for optimal growth of ARCaP cells in vitro. The medium is serum-free and should be supplemented with 5% heat-inactivated fetal bovine serum. It is a bicarbonate buffer, when equilibrated in an incubator (with 5%CO₂/95% air), its pH is 7.4.

Mice can be kept for an appropriately long period of time, e.g., 2 weeks, for tumors to develop to about 100mm³ volume of. Probe solutions for injection will be prepared in sterile phosphate buffered saline (PBS) (probe DZ-1-Lys-DOTA⁶⁸ radioactive dose of Ga complex). As seen by one of ordinary skill, mice can be injected intravenously (e.g., through the tail vein) using an appropriate volume (e.g., a volume of 100-150 µl) of the imaging probe and then under inhalation anesthesia (2% isoflurane in oxygen) Procedures for blood collection, probe biodistribution, and fluorescence and PET imaging will be apparent to those of ordinary skill.

As a first optional step, the Ga-68-DZ-1-Lys-DOTA complex of this example can be administered in this example to detect the primary tumor, and/or can be used to detect any residual metastatic tumors or secondary tumors thereof, for example, after surgical removal of the primary tumor.

The first and/or one or more secondary tumors or metastases of the first or one or more secondary tumors can be treated with the Lu-177 or Y-90 complexes described herein, optionally with Ga-68 for parallel detection of residual metastatic tumors/tumors. Optionally, a first tumor treatment may be performed subsequently, or a second or further tumor treatment if the first tumor treatment has been performed. Image-guided radiotherapy may be performed using the Lu-177 or Y-90 complies of this example. The complexes described herein are particularly advantageous for imaging, therapy, and/or image-guided therapy of prostate cancer, including advantages of, for example, improved serum stability, reduced toxicity, and significantly reduced metastatic bone tumor formation.

### Example 6: Image-guided therapy of brain tumors including glioblastoma

To determine tumor inhibitory effect and/or animal survival and/or body weight, etc., experiments can be performed substantially the same as described in Example 2 above. For example, to assess tumor suppressive effects, nude mice can be subcutaneously inoculated with human brain cancer cells (e.g., neuroblastoma, astrocytoma, or glioblastoma cells, publicly available from the ATCC). Subsequently, the subcutaneous tumor was ~100 mm³ in size of nude mice can be randomly divided into groups (e.g., n=5 mice per group). To assess efficacy on animal survival, therapeutic efficacy, efficacy and toxicity can be determined by inoculating human brain cancer cells in the cranium of the nude mice. One week after inoculation, the test mice were randomized into groups (e.g., n=5 mice per group).

Probe solutions for injection can be prepared in sterile PBS buffer. Imaging probes (in PBS volume, e.g., 100-150 µl) can be injected into mice intravenously (through the tail vein) for blood collection, probe biodistribution, fluorescence, and PET imaging, e.g., through the mouse tail vein, e.g., during inhalation injections are performed under anesthesia (2% isoflurane in oxygen) using a syringe (e.g., a 1/2 cc U-100 insulin syringe). A radioactive dose, also known as a probe, can be DZ-1-Lys-DOTA⁶⁸ Ga complexes, the formation of which will be apparent to the ordinarily skilled artisan and described above.

One or more therapeutic administrations (optionally before or after administration of one or more imaging steps) can generally be performed as described in Example 5 above, with the following modifications: probe solutions for injection can be prepared in sterile PBS buffer. Imaging probes (100-150 µl in PBS volume) for blood collection, probe biodistribution, fluorescence, and PET imaging can be carried out in mice, e.g., by intravenous injection, e.g., through the mouse tail vein, e.g., under anesthesia (2% isoflurane in oxygen) using a syringe (e.g., a 1/2 cc U-100 insulin syringe). The radioactive dose of the probe may be DZ-1-Lys-DOTA⁶⁸Ga complex.

In the first step, a brain tumor inoculated in the subcutaneous space is optionally surgically excised after detection using the Ga-68 complex described herein. In the second step, residual disease can be determined postoperatively and/or within the postoperative interval to detect recurrence using the Ga-68 complex of this example. Treatment with the Lu-177 or Y-90 complex can be initiated after detection of residual or recurrent metastatic tumors or tumors.

In animals with intracranial brain tumors, tumor formation can be detected with the Ga-68 complexes described herein in the first step, ensuring that the complexes cross the blood-brain-barrier. In the second step, brain tumors can be treated with Lu-177 or Y-90 complexes to prevent or slow tumor growth, prevent or delay animal death, and/or cause tumor shrinkage.

The complexes of this example are particularly advantageous for imaging, therapy, and/or image-guided therapy of glioblastoma, including, for example, improving serum stability while maintaining the ability to cross the blood-brain-barrier to improve animal survival.

### Example 7: Testing the specificity and tumor tissue penetration of the complex by imaging methods such as PET

The specificity and tumor tissue permeability of the dye-chelator complexes described herein can be determined by any suitable conventional method, including, for example, PET/CT imaging, e.g., using suitable animal models, which will be apparent to those of ordinary skill. For example, mammalian models, such as mouse models, particularly xenograft mammalian models with the cancer of interest, such as mouse prostate cancer xenograft models, can be used to test conjugates by imaging, particularly PET imaging. Other mammalian models can be used, as will be apparent to the ordinarily skilled artisan (e.g., rat, rabbit, dog, monkey, and etc.).

The radioactive metal complexes of this example can be administered in a manner that will be apparent to one of ordinary skill. For example and without limitation, intraperitoneal administration can be administered to a single mammal (mice), e.g., in groups of five, by injection of 300-500 µCi of the complex. Transaxial microPET images can be acquired periodically, e.g., 12, 24, and 48 hours after probe injection, i.e., at the composite injection (pi) time point. Standardized uptake value (SUV) analysis can be performed on xenograft cancer and muscle of individual mice as determined by CT scan, and tumor-to-muscle ratios can be calculated for each group at these time points, as will be apparent to the ordinarily skilled artisan.

The complex may provide one or more advantages, including enhanced specificity, as shown by PET imaging, such as increased standardized uptake value (SUV) in tumors, enhanced tumor retention and/or tumor penetration, as shown by PET/CT shown. For example, the tumor-to-muscle ratio can be about 8:1 after about 24 hours of dosing, and at about 36 to about 48 hours or longer after dosing, due to prolonged retention of the conjugate in tumor tissue. The complex can significantly increase by about 20% or more (10:1), about 30% or more, about 40% or more, about 50% or more, about 60% or more, about 70% or more, about 80% or more, about 90% or more, about 100% or more, and about 120% or more (20:1).

### Example 8: Image registration and analysis of PET/CT images, determination of SUV

PET/CT images can be processed according to standard protocols, which will be apparent to those of ordinary skill. Pixel-level standardized uptake values (SUVs) for PET can be calculated as pixel-level activity divided by the product of the injected dose and the subject's body weight. By image registration, anatomically overlapping the tumor with the corresponding CT image, the tumor target can be delineated as 40% of the SUV maximum.

### Example 9 - Determination of blood clearance and organ distribution of the complex

After anesthesia (e.g., by administration of 2-3% isoflurane), an appropriately sized group of mammals: 5 mice can be administered with one or more complexes, e.g., radiolabeled, by an appropriate method of administration. DZ-1-Lys-DOTA (DZ-1-Lys-DOTA-¹⁷⁷LuCl₃, DZ-1-Lys-DOTA-⁹⁰YCl₃, DZ-1-Lys-DOTA-⁶⁸GaCl₃) is injected into an appropriately sized group of mammals at a dose of approximately 5 µCi per mouse, via an appropriate vein such as the tail vein. Blood samples, e.g., retro-orbital blood samples of sufficient volume (e.g., about 25 microliters), can be collected periodically at suitable time points, e.g., 5, 15, 30, 60, 180, 360 minutes, and 24 hours after injection of the complex. The radioactivity of all samples can be counted as understood by one of ordinary skill, for example in a gamma counter (e.g., 1480Wizard^{™}, Perkin-Elmer^{™}, Waltham, MA), then the results were normalized and plotted versus the injection time, and a nonlinear regression analysis was performed to obtain the half-life time in blood, e.g., in hours. Experimental animals: mice, which can be sacrificed immediately after the last blood collection. Tumors and organs (e.g., heart, liver, lung, kidney, small intestine, stomach, bone, muscle, spleen, and skin) can be collected separately and organ-specific radioactivity and/or half-life and/or organ residence time can be performed substantially as described above Determination. The results showed that DZ-1-Lys-DOTA-Lu-177, DZ-1-Lys-DOTA-Y-90, DZ-1-Lys-DOTA-Ga-68 had good blood and organ clearance. When comparing these complexes with other complexes, especially with the DZ-1-Lys-DOTA-Cu-64 complex, excellent clearance rates were shown.

### Clearance rate of DZ-1-Lys-DOTA-⁶⁸Ga in blood

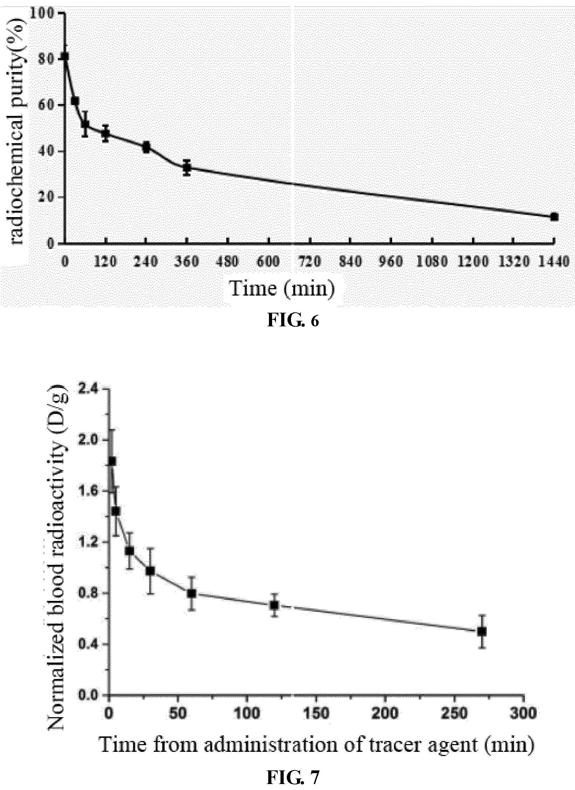

DZ-1-Lys-DOTA-⁶⁸Ga (50 µCi) was injected into the tail vein of the Balb/C mice (n=6, approximately 20 g). About 10 µL of blood was collected from the lateral tail vein into capillaries 2 minutes, 5 minutes, 15 minutes, 30 minutes, 1 hour, 2 hours and 4.5 hours after probe injection, and the capillaries were placed in pre-weighed test tubes to measure the weight and radioactivity of the drawn blood. The radioactivity of each sample was measured by a gamma counter. The radioactivity decayed with the injection time of the probe, was normalized with the injection dose and blood weight, and expressed as a percentage of the injected dose per gram of blood, and the results are shown in FIG. 7 (50 µCi/probes was intravenously injected into each animal).

### Clearance rate of DZ-1-Lys-DOTA-177 Lu in blood

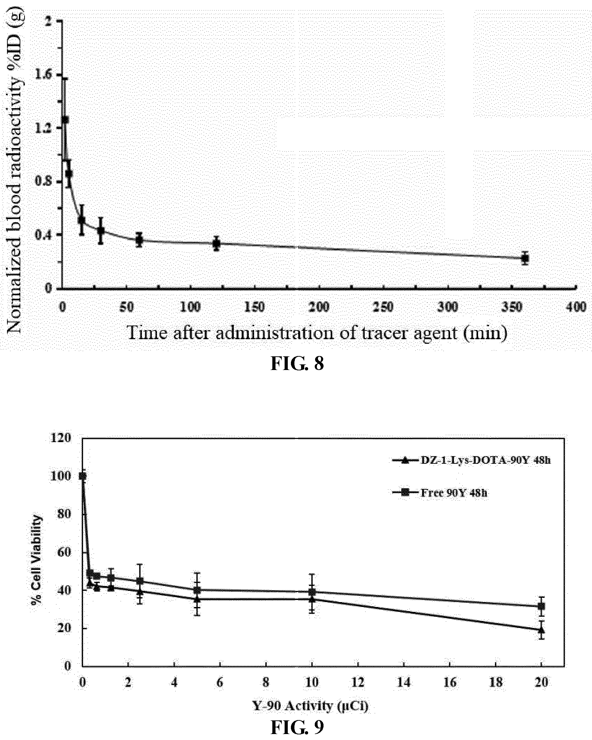

DZ-1-Lys-DOTA-¹⁷⁷Lu (50 µCi) was injected into the tail vein of the Balb/C mice (n=6, approximately 20 g). About 10 µL of blood was collect from the lateral tail vein into the capillaries 2 minutes, 5 minutes, 15 minutes, 30 minutes, 1 hour, 2 hours and 6 hours after probe injection, and the capillaries were placed in pre-weighed test tubes to measure the weight and radioactivity of the drawn blood. The radioactivity of each sample was measured by a gamma counter, the radioactivity decays with the injection time of the probe, was normalized with the injection dose and blood weight, and expressed as a percentage of the injected dose per gram of blood, and the results are shown in FIG. 8 (50 µCi/probe was intravenously injected into each animal).

### Cytotoxicity of DZ-1-Lys-DOTA-⁹⁰ Y

MDA-MB-231 breast cancer cells were pre-inoculated into 96-well plates (10⁴ cells/well) and placed in a 5% CO₂ incubator at 37°C 5%CO₂ until the cells were completely adherent. Serially diluted DZ-1-Lys-DOTA-⁹⁰Y (100 µL) was to each well and 3 replicate wells for each concentration point. After 48 h of treatment, the supernatant was discarded and 10 µL of CCK-8 solution and 100 µL of culture medium were added to each well, incubation was continued in the incubator for further 1 h and the absorbance of each well was measured at 450 nm using a microplate reader, and the results are shown in the FIG. 9. As shown in 9. DZ-1-Lys-DOTA-⁹⁰Y killed MDA-MB-231 triple-negative breast cancer cells, and DZ-1-Lys-DOTA-90 Y killed breast cancer cells more effectively when compared to ⁹⁰Y.

## Claims

1. A pharmaceutical composition comprising a first complex and a second complex of DZ-1-Lys-DOTA conjugate with a radioactive metal M, **characterized in that** said DZ-1-Lys-DOTA conjugate comprises a heptamethyl carbocyanine dye group coupled with a DOTA group through a lysine cross-linking agent, and the molecular formula of the complex is as follows: 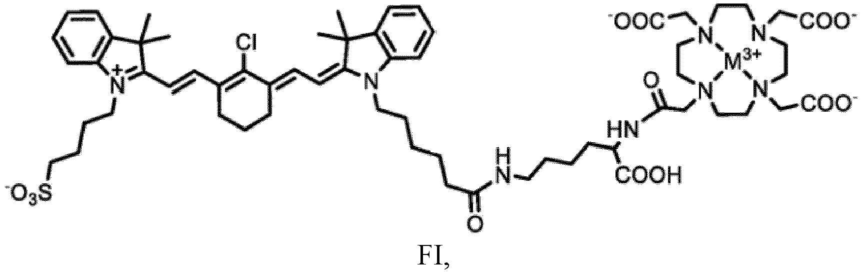
wherein the DOTA group is complexed with the radioactive metal M; and;
wherein the radioactive metal in the first complex is gallium-68, and the radioactive metal in the second complex is lutetium-177 or yttrium-90.

2. The pharmaceutical composition according to claim 1, **characterized in that** the pharmaceutical composition further comprises one or more pharmaceutical excipients.

3. The pharmaceutical composition according to claim 1, **characterized in that** the complex is formed by mixing and complexing the DZ-1-Lys-DOTA conjugate with the radioactive metals, and with one or more reagents, buffers or excipients;
wherein the DZ-1-Lys-DOTA conjugate has the molecular structure of formula FII:

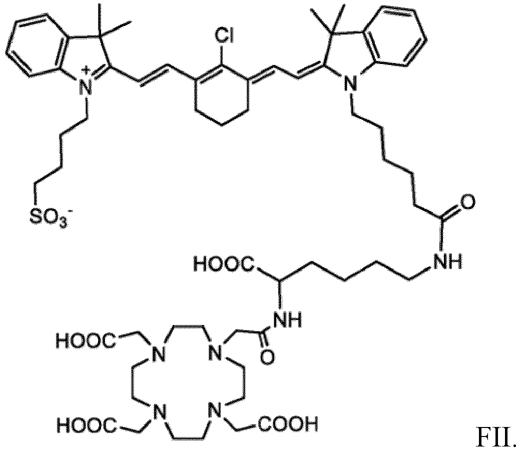

4. The pharmaceutical composition according to claim 1 for use in the image-guided therapy of cancer, **characterized in that** the first complex and the second complex of the DZ-1-Lys-DOTA conjugate having a molecular structure FI are co-administered to a subject with cancer.

5. The pharmaceutical composition for use according to claim 4, **characterized in that** the cancer comprises precancerous and cancer cells or tissues, tumors and metastatic tumors thereof, primary and secondary tumors.

6. The pharmaceutical composition for use according to claim 4, **characterized in that** the cancer comprises brain cancer, brain tumors and metastatic tumors thereof.

7. The pharmaceutical composition for use according to claim 4, **characterized in that** the treatment of the cancer is a radiation therapy.

8. The pharmaceutical composition for use according to claim 7, **characterized in that** a radioactive amount of the complex during the radiation therapy is less than 50 µCi.

9. The pharmaceutical composition for use according to claim 7, **characterized in that** a dose of the complex is less than 250 kBq/kg in the radiation therapy.

10. The pharmaceutical composition for use according to claim 7, **characterized in that** the radiation therapy is paired with positron emission tomography or SPECT and/or provides image-guided therapy.

11. The pharmaceutical composition for use according to claim 4, **characterized in that** a route of administration of the pharmaceutical composition is oral, intravenous, interstitial, intraperitoneal, subcutaneous or intramuscular injection, and/or by brachytherapy.

12. The pharmaceutical composition according to claim 1, **characterized in that** the pharmaceutical composition contains 0.01% to 99% of the first complex and the second complex.

## Patentansprüche

1. Eine pharmazeutische Zusammensetzung, die einen ersten Komplex und einen zweiten Komplex eines DZ-1-Lys-DOTA-Konjugats mit einem radioaktiven Metall M umfasst, **dadurch gekennzeichnet, dass** das DZ-1-Lys-DOTA-Konjugat eine Heptamethylcarbocyaninfarbstoffgruppe umfasst, die über ein Lysin-Vernetzungsmittel mit einer DOTA-Gruppe gekoppelt ist, und die Molekülformel des Komplexes wie folgt lautet: 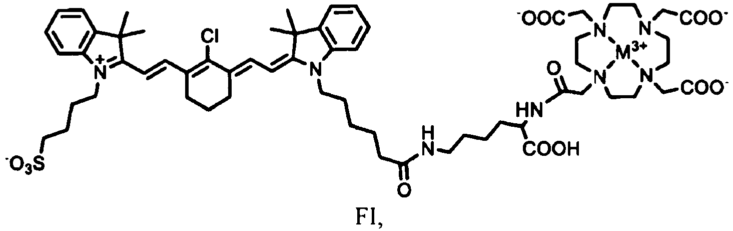
wobei die DOTA-Gruppe mit dem radioaktiven Metall M komplexiert ist; und;
wobei das radioaktive Metall im ersten Komplex Gallium-68 ist und das radioaktive Metall im zweiten Komplex Lutetium-177 oder Yttrium-90 ist.

2. Die pharmazeutische Zusammensetzung gemäß Anspruch 1, **dadurch gekennzeichnet, dass** die pharmazeutische Zusammensetzung ferner einen oder mehrere pharmazeutische Hilfsstoffe umfasst.

3. Die pharmazeutische Zusammensetzung gemäß Anspruch 1, **dadurch gekennzeichnet, dass** der Komplex durch Mischen und Komplexieren des DZ-1-Lys-DOTA-Konjugats mit den radioaktiven Metallen und mit einem oder mehreren Reagenzien, Puffern oder Hilfsstoffen gebildet wird;
wobei das DZ-1-Lys-DOTA-Konjugat die Molekülstruktur der Formel FII aufweist:

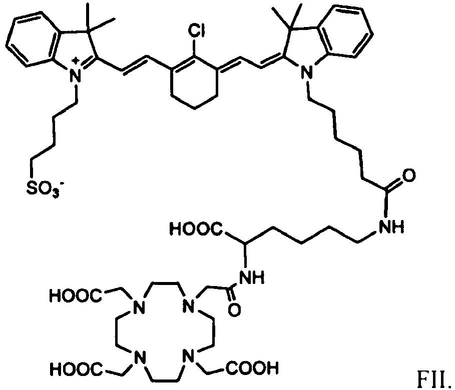

4. Die pharmazeutische Zusammensetzung gemäß Anspruch 1 zur Verwendung in der bildgesteuerten Therapie von Krebs, **dadurch gekennzeichnet, dass** der erste Komplex und der zweite Komplex des DZ-1-Lys-DOTA-Konjugats mit einer Molekülstruktur FI einem Subjekt mit Krebs gemeinsam verabreicht werden.

5. Die pharmazeutische Zusammensetzung zur Verwendung gemäß Anspruch 4, **dadurch gekennzeichnet, dass** der Krebs präkanzeröse Zellen und Krebszellen oder Gewebe, Tumore und metastatische Tumore davon, und primäre und sekundäre Tumore umfasst.

6. Die pharmazeutische Zusammensetzung zur Verwendung gemäß Anspruch 4, **dadurch gekennzeichnet, dass** der Krebs Hirnkrebs, Hirntumore und deren metastatische Tumore umfasst.

7. Die pharmazeutische Zusammensetzung zur Verwendung gemäß Anspruch 4, **dadurch gekennzeichnet, dass** die Behandlung von Krebs eine Strahlentherapie ist.

8. Die pharmazeutische Zusammensetzung zur Verwendung gemäß Anspruch 7, **dadurch gekennzeichnet, dass** eine radioaktive Menge des Komplexes während der Strahlentherapie weniger als 50 µCi beträgt.

9. Die pharmazeutische Zusammensetzung zur Verwendung gemäß Anspruch 7, **dadurch gekennzeichnet, dass** eine Dosis des Komplexes weniger als 250 kBq/kg in der Strahlentherapie beträgt.

10. Die pharmazeutische Zusammensetzung zur Verwendung gemäß Anspruch 7, **dadurch gekennzeichnet, dass** die Strahlentherapie mit Positronen-Emissions-Tomographie oder SPECT kombiniert wird und/oder bildgesteuerte Therapie bereitstellt.

11. Die pharmazeutische Zusammensetzung zur Verwendung gemäß Anspruch 4, **dadurch gekennzeichnet, dass** ein Verabreichungsweg der pharmazeutischen Zusammensetzung eine orale, intravenöse, interstitielle, intraperitoneale, subkutane oder intramuskuläre Injektion ist und/oder durch Brachytherapie erfolgt.

12. Die pharmazeutische Zusammensetzung gemäß Anspruch 1, **dadurch gekennzeichnet, dass** die pharmazeutische Zusammensetzung 0,01% bis 99% des ersten Komplexes und des zweiten Komplexes beinhaltet.

## Revendications

1. Composition pharmaceutique comprenant un premier complexe et un second complexe d'un conjugué DZ-1-Lys-DOTA avec un métal radioactif M, **caractérisée en ce que** ledit conjugué DZ-1-Lys-DOTA comprend un groupement colorant heptaméthylcarbocyanine couplé à un groupement DOTA par l'intermédiaire d'un agent de réticulation lysine, et la formule moléculaire du complexe est la suivante : 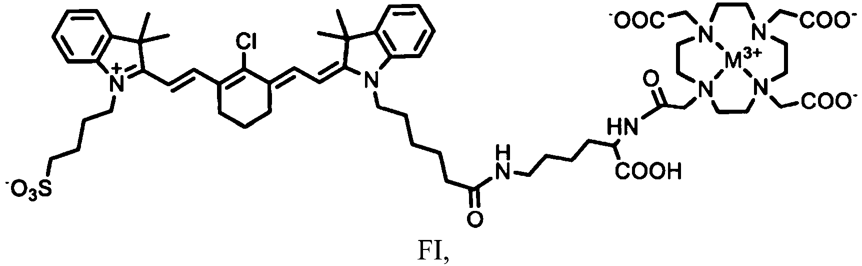
dans laquelle le groupement DOTA est complexé au métal radioactif M ; et ;
dans laquelle le métal radioactif dans le premier complexe est le gallium 68, et le métal radioactif dans le second complexe est le lutécium 177 ou l'yttrium 90.

2. Composition pharmaceutique selon la revendication 1, **caractérisée en ce que** la composition pharmaceutique comprend en outre un ou plusieurs excipients pharmaceutiques.

3. Composition pharmaceutique selon la revendication 1, **caractérisée en ce que** le complexe est formé en mélangeant et complexant le conjugué DZ-1-Lys-DOTA avec les métaux radioactifs, et avec un ou plusieurs réactifs, tampons ou excipients ;
dans lequel le conjugué DZ-1-Lys-DOTA a la structure moléculaire de formule FII :

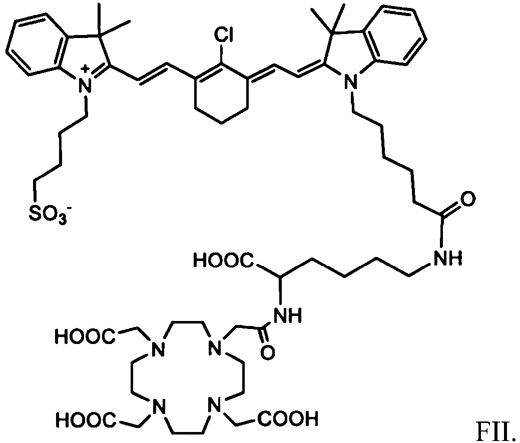

4. Composition pharmaceutique selon la revendication 1 pour une utilisation dans la thérapie guidée par imagerie du cancer, **caractérisée en ce que** le premier complexe et le second complexe du conjugué DZ-1-Lys-DOTA ayant une structure moléculaire FI sont co-administrés à un sujet atteint de cancer.

5. Composition pharmaceutique pour une utilisation selon la revendication 4, **caractérisée en ce que** le cancer comprend des cellules ou des tissus précancéreux et cancéreux, des tumeurs et des tumeurs métastatiques de ceux-ci, des tumeurs primaires et secondaires.

6. Composition pharmaceutique pour une utilisation selon la revendication 4, **caractérisée en ce que** le cancer comprend un cancer du cerveau, des tumeurs cérébrales et des tumeurs métastatiques de ceux-ci.

7. Composition pharmaceutique pour une utilisation selon la revendication 4, **caractérisée en ce que** le traitement du cancer est une radiothérapie.

8. Composition pharmaceutique pour une utilisation selon la revendication 7, **caractérisée en ce qu'**une quantité radioactive du complexe pendant la radiothérapie est inférieure à 50 µCi.

9. Composition pharmaceutique pour une utilisation selon la revendication 7, **caractérisée en ce qu'**une dose du complexe est inférieure à 250 kBq/kg dans la radiothérapie.

10. Composition pharmaceutique pour une utilisation selon la revendication 7, **caractérisée en ce que** la radiothérapie est couplée à la tomographie par émission de positons ou SPECT et/ou fournit une thérapie guidée par imagerie.

11. Composition pharmaceutique pour une utilisation selon la revendication 4, **caractérisée en ce qu'**une voie d'administration de la composition pharmaceutique est la voie orale, une injection intraveineuse, interstitielle, intrapéritonéale, sous-cutanée ou intramusculaire, et/ou par curiethérapie.

12. Composition pharmaceutique selon la revendication 1, **caractérisée en ce que** la composition pharmaceutique contient 0,01 % à 99 % du premier complexe et du second complexe.
